(19) Europäisches Patentamt
European Patent Office
Office européen des brevets

(11) Publication number: 0 321 175 A1

(12) EUROPEAN PATENT APPLICATION

(21) Application number: 88311762.4

(22) Date of filing: 13.12.88

(51) Int. Cl.4: C07D 211/76 , C07D 207/263 , C07D 213/64 , C07C 103/78 , C07C 103/38 , A61K 31/445 , A61K 31/40 , A61K 31/44 , A61K 31/165

Claims for the following Contracting State: ES.

The title of the invention has been amended (Guidelines for Examination in the EPO, A-III, 7.3).

(30) Priority: 14.12.87 GB 8729141
09.08.88 GB 8818877
09.08.88 GB 8818878

(43) Date of publication of application: 21.06.89 Bulletin 89/25

(84) Designated Contracting States: AT BE CH DE ES FR GB GR IT LI LU NL SE

(71) Applicant: BEECHAM GROUP PLC
Beecham House Great West Road
Brentford Middlesex TW8 9BD(GB)

(72) Inventor: Tedder, John Martin Beecham Pharmaceuticals
Great Burgh Yew Tree Bottom Road
Epsom Surrey KT18 5XQ(GB)
Inventor: Pinto, Ivan Leo Beecham Pharmaceuticals
Great Burgh Yew Tree Bottom Road
Epsom Surrey KT18 5XQ(GB)
Inventor: Edge, Colin Michael Beecham Pharmaceuticals
Great Burgh Yew Tree Bottom Road
Epsom Surrey KT18 5XQ(GB)

(74) Representative: Rutter, Keith et al
Beecham Pharmaceuticals Great Burgh Yew Tree Bottom Road
Epsom Surrey KT18 5XQ(GB)

(54) **Indane derivatives.**

(57) A compound of formula (I):

EP 0 321 175 A1

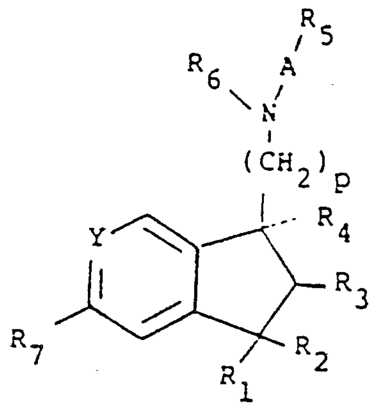

or, where appropriate, a pharmaceutically acceptable salt thereof, or a pharmaceutically acceptable solvate thereof

wherein

A represents >C=X, wherein

X represents oxygen or sulphur, or A represents a bond;

Y represents N or $N^{+}$-$O^{-}$ or a moiety $CR_8$,

and the other substituents are defined as in claim 1;

a process for the preparation of such a compound, a pharmaceutical composition comprising such a compound and the use of such a compound and composition in medicine.

## NOVEL COMPOUNDS

This invention relates to certain novel compounds, in particular to novel indane derivatives having smooth muscle relaxant activity, to processes for their preparation, to compositions containing such compounds and to the use of such compounds and compositions in medicine.

EP-A-76075, 93535, 95316, 107423, 120426, 126311, 126350, 126367 and 138134 describe certain benzopyran derivatives having inter alia antihypertensive activity. EP-A-176689 also discloses that certain benzopyran derivatives are useful for the treatment of inter alia disorders of the respiratory system.

European Patent Application, Publication Number 0,272,982 discloses certain indane derivatives as having diuretic activity and antiarrhythmic, cerebral anti-ischaemic and hypotensive properties and also discloses indane intermediates for preparing such compounds. Belgian patent No. 667,486 and French Patent No. 1,462,244 disclose certain indanes as having analgesic, local anaesthetic and CNS-depressant activity. Netherlands Patent No. 68,15088 discloses certain indane derivatives as having hypoglycaemic activity.

A group of novel indane derivatives has now been discovered which surprisingly has smooth muscle relaxant activity, and such compounds are therefore potentially useful as bronchodilators in the treatment of disorders of the respiratory tract, such as reversible airways obstruction and asthma, and also in the treatment of hypertension. Such compounds are also indicated to be of potential use in the treatment of disorders associated with smooth muscle contraction of the gastro-intestinal tract, uterus or the urinary tract including the ureter. Such disorders respectively include irritable bowel syndrome and diverticular disease; premature labour; incontinence; renal cholic and disorders associated with the passage of kidney stones. They are also indicated as of potential use in the treatment of cardiovascular disorders other than hypertension, such as congestive heart failure, angina, peripheral vascular disease and cerebral vascular disease; and also in the treatment and/or prophylaxis of disorders associated with pulmonary hypertension and of disorders associated with right heart failure.

Accordingly, the present invention provides a compound of formula (I):

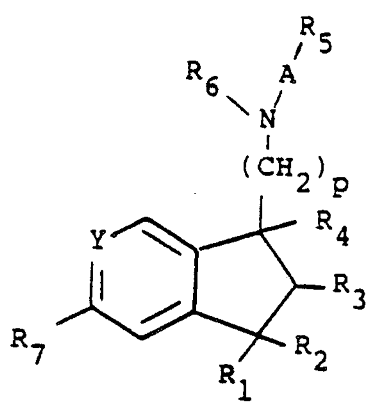

(I)

or, where appropriate, a pharmaceutically acceptable salt thereof, or a pharmaceutically acceptable solvate thereof
wherein:
A represents >C=X, wherein
X represents oxygen or sulphur, or A represents a bond;
Y represents N or $N^+$-$O^-$ or a moiety $CR_8$ wherein
$R_8$ is as defined below;
$R_1$ and $R_2$ independently represent hydrogen or alkyl; or
$R_1$ and $R_2$ together represent a $C_{2-7}$ polymethylene moiety;
$R_3$ represents hydrogen, hydroxy, alkoxy or acyloxy and $R_4$ is hydrogen or $R_3$ and $R_4$ together represent a bond;
when A represents >C=X, then $R_5$ is hydrogen;
alkyl optionally substituted by one or more groups or atoms selected from halogen, hydroxy, alkoxy,

alkoxycarbonyl, carboxy or an ester or amide thereof, amino, monoalkylamino or dialkylamino; alkenyl; amino optionally substituted by an alkyl or alkenyl group or by an alkanoyl group optionally substituted by up to three halo atoms, by a phenyl group optionally substituted by alkyl, alkoxy or halogen; substituted or unsubstituted aryl or substituted or unsubstituted heteroaryl; and

$R_6$ represents hydrogen or alkyl;

or $R_5$ and $R_6$ together represent a linking chain of formula $-A^1-A^2-$, $A^1$ being attached to the nitrogen atom of the moiety -N-A- and $A^2$ being attached to the group A on the said moiety, and wherein $A^1$ represents a substituted or unsubstituted methylene group, $A^2$ represents 2 or 3 linking members, one of the linking members optionally representing O, S or NR and the other linking members each independently representing a substituted or unsubstituted methylene group; R represents hydrogen, alkyl, alkanoyl, phenyl $C_{1-4}$-alkyl, arylcarbonyl wherein the aryl group may be substituted or unsubstituted;

or R is mono- or bi-cyclic-heteroarylcarbonyl; when A represents a bond, then $R_5$ and $R_6$ together with the nitrogen atom to which they are attached, form an unsaturated heterocyclic ring having 5 to 7 ring atoms, which ring atoms comprise up to 2 further nitrogen atoms and a carbon atom, the carbon atom being substituted with either an oxo group or a thioxo group the remaining ring atoms being substituted or unsubstituted;

$R_7$ and $R_8$ are each independently selected from the class of hydrogen, substituted or unsubstituted alkyl, alkoxy, $C_{3-8}$ cycloalkyl, hydroxy, nitro, cyano, halo, formyl, carboxy, a group of formula $R^aT^1-$, $R^bR^cNT-$, $R^aT^2NH-$, $R^dCO.O-$, $R^dCS.O-$, $R^d(OH)CH-$, $R^d(SH)CH-$, $R^dC(=N.OH)-$, $R^dC(=N.NH_2)-$ or alkenyl optionally substituted by alkylcarbonyl, nitro or cyano, providing that when Y is $CR_8$ then at least one of $R_7$ or $R_8$ is not hydrogen;

$R^a$ represents $R^d$ or $R^dO-$ and $R^d$ represents substituted or unsubstituted alkyl, substituted or unsubstituted aryl or substituted or unsubstituted heteroaryl, optional substituents for $R^d$ being up to 3 substituents selected from alkyl, alkoxy, halo, haloalkyl, nitro and cyano;

$R^b$ and $R^c$ each independently represent hydrogen, alkyl or alkylcarbonyl;

T represents a bond or $T^1$, $T^1$ represents -CS- or $T^2$ and $T^2$ represents -CO-, -SO- or $-SO_2-$; and

p represents zero or an integer 1.

Suitable substituents for any aryl or heteroaryl group represented by $R_5$ include one or more groups or atoms selected from alkyl, alkoxy, hydroxy, halogen, fluoroalkyl, nitro, cyano, carboxy or an ester thereof, alkylcarbonyloxy, amino, monoalkylamino, dialkylamino, aminocarbonyl, monoalkylaminocarbonyl or dialkylaminocarbonyl.

When the linking chain $-A^1-A^2-$ comprises substituted methylene groups it is favoured if one or two of methylene groups are substituted, in particular it is favoured if the methylene group represented by $-A^1-$ is substituted.

Suitable substituents for any methylene group in $-A^1-A^2-$ include alkyl groups, especially methyl or ethyl and in particular methyl.

In one particular aspect when A represents >C=X, the linking chain $-A^1-A^2-$ (and thus $R_5$ and $R_6$ together) represent a moiety of formula $-CH_2-(CH_2)_n-Z-(CH_2)_m-$wherein m and n are 0 to 2 such that m + n is 1 or 2 and Z is $CH_2$, O, S or NR wherein R is as defined above.

Suitably R represents hydrogen, $C_{1-9}$ alkyl, $C_{2-7}$ alkanoyl, phenyl-$C_{1-4}$-alkyl, naphthylcarbonyl, phenylcarbonyl or benzylcarbonyl optionally substituted in the phenyl or naphthyl ring by one or two of $C_{1-6}$ alkyl, $C_{1-6}$ alkoxy or halogen; or R is mono- or bi-cyclic- heteroarylcarbonyl.

Suitably, when Y is N or $N^+-O^-$, $R_7$ is hydrogen.

Suitably, when Y is $CR_8$, then one of $R_7$ and $R_8$ is hydrogen.

In particular, when Y is $CR_8$, one of $R_7$ and $R_8$ is hydrogen and the other is selected from substituted or unsubstituted alkyl, alkoxy, $C_{3-8}$ cycloalkyl, hydroxy, nitro, cyano, halo, formyl, carboxy, a group of formula $R^aT^1-$, $R^bR^cNT-$, $R^aT^2NH-$, $R^dCO.O-$, $R^dCS.O-$, $R^d(OH)CH-$, $R^d(SH)CH-$, $R^dC(=N.OH)-$, $R^dC(=N.NH_2)-$ or alkenyl optionally substituted by alkylcarbonyl, nitro or cyano, wherein $R^a$, $R^b$, $R^c$, T, $T^1$ and $T^2$ are as defined in relation to formula (I).

Also to be mentioned are those compounds wherein one of $R_7$ and $R_8$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkyl-thiocarbonyl, $C_{1-6}$ alkoxy-thiocarbonyl, $C_{1-6}$ alkyl-thiocarbonyloxy, $C_{1-6}$ alkyl-thiomethyl, formyl or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino $C_{1-6}$ alkoxysulphinylamino or $C_{1-6}$ alkoxysulphonylamino or ethenyl terminally substituted by $C_{1-6}$ alkylcarbonyl, nitro or cyano, or $-C(C_{1-6}$ alkyl)NOH or $-C(C_{1-6}$ alkyl)$NNH_2$.

In a further particular aspect, $R_7$ represents hydrogen and $R_8$ represents alkyl or $C_{3-8}$ cycloalkyl.

Suitably, one of $R_7$ and $R_8$ represents nitro, cyano or alkylcarbonyl and the other represents a different group selected from nitro, cyano, halo, alkylcarbonyl, alkoxy, especially methoxy, mono- or di-alkylamino or mono- or di-alkylcarbonylamino. For example, one of $R_7$ and $R_8$ may represent nitro, cyano or alkylcarbonyl and the other may represent alkoxy, especially methoxy, amino, mono- or di-alkylamino or mono- or di-alkylcarbonylamino.

When $R_7$ or $R_8$ represents a substituted alkyl group, suitable optional substituents include one or more of halogen, hydroxy, alkoxy, thiol, nitro, cyano and alkylcarbonyl, especially halogen.

In one particular aspect, the present invention provides a compound of formula (I), or, where appropriate, a pharmaceutically acceptable salt thereof, or a pharmaceutically acceptable solvate thereof wherein:

A represents >C=X, wherein

X represents oxygen or sulphur, or A represents a bond;

Y represents N or $N^{+}$-$O^{-}$ or a moiety $CR_8$ wherein

$R_8$ is as defined below;

$R_1$ and $R_2$ independently represent hydrogen or $C_{1-6}$ alkyl; or $R_1$ and $R_2$ together represent a $C_{2-7}$ polymethylene moiety;

$R_3$ represents hydrogen, hydroxy, $C_{1-6}$ alkoxy or $C_{1-7}$ acyloxy and $R_4$ is hydrogen or $R_3$ and $R_4$ together represent a bond;

when A represents >C=X, then $R_5$ is hydrogen; $C_{1-6}$ alkyl optionally substituted by halogen, hydroxy, $C_{1-6}$ alkoxy, $C_{1-6}$ alkoxycarbonyl, carboxy or amino optionally substituted by one or two independent $C_{1-6}$ alkyl groups; or $C_{2-6}$ alkenyl; amino optionally substituted by a $C_{1-6}$ alkyl or $C_{2-6}$ alkenyl group or by a $C_{1-6}$ alkanoyl group optionally substituted by up to three halo atoms, by a phenyl group optionally substituted by $C_{1-6}$ alkyl, $C_{1-6}$ alkoxy or halogen; or aryl or heteroaryl, either being optionally substituted by one or more groups or atoms selected from the class of $C_{1-6}$ alkyl, $C_{1-6}$ alkoxy, hydroxy, halogen, trifluoromethyl, nitro, cyano, $C_{1-12}$ carboxylic acyl, or amino or aminocarbonyl optionally substituted by one or two $C_{1-6}$ alkyl groups; and

$R_6$ represents hydrogen or $C_{1-6}$ alkyl;

or $R_5$ and $R_6$ together represent $-CH_2-(CH_2)_n-Z-(CH_2)_m-$wherein m and n are 0 to 2 such that m + n is 1 or 2 and Z is $CH_2$, O, S or NR wherein R is hydrogen, $C_{1-9}$ alkyl, $C_{2-7}$ alkanoyl, phenyl $C_{1-4}$- alkyl, naphthylcarbonyl, phenylcarbonyl or benzylcarbonyl optionally substituted in the phenyl or naphthyl ring by one or two of $C_{1-6}$ alkyl, $C_{1-6}$ alkoxy or halogen; or R is mono- or bi-cyclic- heteroarylcarbonyl;

when A represents a bond, then $R_5$ and $R_6$ together with the nitrogen atom to which they are attached, form an unsaturated heterocyclic ring having 5 to 7 ring atoms, which ring atoms comprise up to 2 further nitrogen atoms and a carbon atom, the carbon atom being substituted with either an oxo group or a thioxo group the remaining ring atoms being substituted or unsubstituted;

when Y is N or $N^{+}$-$O^{-}$, $R_7$ is hydrogen or, when Y is $CR_8$, either one of $R_7$ and $R_8$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkyl-thiocarbonyl, $C_{1-6}$ alkoxy-thiocarbonyl, $C_{1-6}$ alkyl-thiocarbonyloxy, $C_{1-6}$ alkyl-thiomethyl, formyl or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino $C_{1-6}$ alkoxysulphinylamino or $C_{1-6}$ alkoxysulphonylamino or ethenyl terminally substituted by $C_{1-6}$ alkylcarbonyl, nitro or cyano, or -C-($C_{1-6}$ alkyl)NOH or -C($C_{1-6}$ alkyl)$NNH_2$; or one of $R_7$ and $R_8$ is nitro, cyano or $C_{1-3}$ alkylcarbonyl and the other is methoxy or amino optionally substituted by one or two $C_{1-6}$ alkyl or by $C_{2-7}$ alkanoyl; or $R_7$ is hydrogen and $R_8$ is $C_{1-6}$ alkyl or $C_{3-8}$ cycloalkyl; suitably in such compounds p represents zero; alternatively p may represent the integer 1.

Preferably, when A represents >C=X then X is oxygen.

Preferably, Y represents C-$R_8$ wherein $R_8$ is as defined in relation to formula (I).

Preferably, $R_1$ and $R_2$ are both $C_{1-6}$ alkyl, and in particular $R_1$ and $R_2$ are both methyl.

Further examples of the compounds of formula (I) include those wherein, $R_1$ and $R_2$ both represent hydrogen.

When $R_3$ is alkoxy, suitably $C_{1-6}$ alkoxy, and $R_4$ is hydrogen, preferred examples of $R_3$ include methoxy and ethoxy, of which methoxy is more preferred. When $R_3$ is acyloxy, suitably $C_{1-7}$ acyloxy, and $R_4$ is hydrogen, a preferred class of $R_3$ is unsubstituted carboxylic acyloxy, such as unsubstituted aliphatic acyloxy.

However, it is more preferred that $R_3$ and $R_4$ together are a bond, or that $R_3$ and $R_4$ are both hydrogen,

or in particular, that $R_3$ is hydroxy and $R_4$ is hydrogen.

When $R_5$ represents alkyl it is suitably $C_{1-6}$ alkyl, favoured alkyl groups include methyl, ethyl and n- and iso-propyl, preferably methyl.

A suitable halogen substituent for any alkyl represented by $R_5$, is a fluoro, chloro or bromo substituent; favoured examples include methyl, ethyl or propyl, especially n-propyl, terminally substituted by fluoro, chloro or bromo, for example chloro.

When $R_5$ represents alkyl substituted by hydroxy, favoured examples include methyl or ethyl terminally substituted by hydroxy.

When $R_5$ represents alkyl substituted by alkoxy, a suitable alkoxy group is a methoxy or ethoxy group; favoured examples include methyl or ethyl terminally substituted by methoxy or ethoxy.

When $R_5$ represents alkyl substituted by alkoxycarbonyl, suitably $C_{1-6}$ alkoxycarbonyl, a suitable alkoxycarbonyl group is a methoxycarbonyl or ethoxycarbonyl group; examples include methyl or ethyl terminally substituted by methoxycarbonyl or ethoxycarbonyl.

When $R_5$ represents alkyl substituted by carboxy, favoured examples include methyl or ethyl terminally substituted by carboxy.

When $R_5$ represents alkyl substituted by amino wherein the amino group is optionally substituted by one or two independent alkyl groups, favoured values include a group $(CH_2)_nNR_9R_9{}^1$ where n is 1 to 6, and $R_9$ and $R_9{}^1$are each independently hydrogen or $C_{1-6}$ alkyl; suitable values for n include 1 and 2, in particular 1.

Preferably $R_9$ and $R_9{}^1$ are each independently selected from hydrogen and methyl.

When $R_5$ represents alkenyl, suitably $C_{2-6}$ alkenyl, suitable values include vinyl, prop-1-enyl, prop-2-enyl, 1-methylvinyl, but-1-enyl, but-2-enyl, but-3-enyl, 1-methylenepropyl, or 1-methylprop-2-enyl, in both their E and Z forms where stereoisomerism exists.

When $R_5$ represents optionally substituted amino, suitable optional substituents for the amino group include a methyl; ethyl; propyl; butyl; allyl or a trichloroacetyl group; or a phenyl group optionally substituted by one methyl, methoxy group or one halogen atom, and in particular a phenyl group optionally substituted with amino, methylamino or phenylamino; the phenyl group in the phenylamino substituent being optionally substituted in the phenyl ring by one methyl or methoxy group or one halogen atom.

When $R_5$ represents aryl, favoured examples include phenyl and naphthyl, preferably phenyl.

When $R_5$ represents heteroaryl, suitable heteroaryl groups include 5- or 6-membered monocyclic or 9- or 10-membered bicyclic heteroaryl groups, preferably 5-or 6-membered monocyclic heteroaryl groups.

Preferred 5- or 6-membered monocyclic or 9- or 10-membered bicyclic heteroaryl groups are those containing one, two or three heteroatoms selected from the class of oxygen, nitrogen and sulphur and which, in the case of there being more than one heteroatom, are the same or different.

Suitable 5- or 6-membered monocyclic heteroaryl moieties include furyl, thienyl, pyrrolyl, oxazolyl, thiazolyl, imidazolyl and thiadiazolyl, and pyridyl, pyridazyl, pyrimidyl, pyrazyl and triazyl. Preferred 5- or 6-membered heteroaryl groups include furyl, thienyl, pyrrolyl and pyridyl, in particular 2- and 3-furyl, 2- and 3-pyrrolyl 2- and 3-thienyl, and 2-, 3-and 4-pyridyl.

Suitable 9- or 10-membered bicyclic heteroaryl moieties include benzofuryl, benzothienyl, indolyl and indazolyl, quinolinyl and isoquinolinyl, and quinazolinyl. Preferred 9- or 10- membered bicyclic heteroaryl groups include 2- and 3-benzofuryl, 2- and 3-benzothienyl, and 2- and 3-indolyl, and 2- and 3-quinolinyl.

In any optionally substituted aryl or optionally substituted heteroaryl group, the preferred number of substituents is 1, 2, 3 or 4.

Preferred substituents for any substituted aryl or heteroaryl group include methyl, methoxy, hydroxy, chloro, fluoro, nitro or cyano.

One preferred sub-group of values for $R_5$ is that wherein $R_5$ represents phenyl or naphthyl or a 5- or 6-membered monocyclic or a 9- or 10-membered bicyclic heteroaryl, the phenyl, naphthyl or heteroaryl group being optionally substituted by one, two, three or four groups or atoms selected from the class of alkyl, alkoxy, halogen, fluoroalkyl, nitro or cyano.

When $R_5$ represents optionally substituted phenyl, preferred values include phenyl, 4-substituted phenyl, 3-substituted phenyl, 3,4-disubstituted phenyl and 3, 4, 5-trisubstituted phenyl, for example $R_5$ may suitably represent 4-fluorophenyl.

When $R_5$ represents an optionally substituted 5- or 6-membered monocyclic heteroaryl or an optionally substituted 9- or 10-membered bicyclic heteroaryl group, preferred values include unsubstituted 5- or 6-membered monocyclic heteroaryl or mono-substituted 5-or 6-membered monocyclic heteroaryl or 9- or 10-membered bicyclic heteroaryl, in particular unsubstituted 5- or 6-membered monocyclic heteroaryl or 9- or 10-membered bicyclic heteroaryl.

Preferably, when $R_5$ and $R_6$ together represent a linking chain $-A^1-A^2-$, $A^1$ represents a substituted or

unsubstituted methylene group and $A^2$ represents a $-CH_2CH_2-$ or $-CH_2CH_2CH_2-$ group, for example $-CH_2CH_2-$.

When $A^1$ represents a substituted methylene group it is preferably substituted by an alkyl group especially a methyl group.

In one suitable aspect when $R_5$ and $R_6$ together represent $-A^1-A^2-$ the moiety $R_5.N.CX.R_6$ represents 5′-methylpyrrolidonyl or 6′-methylpiperidonyl, especially 5′-methylpyrrolidonyl.

In a preferred aspect $R_5$ and $R_6$ together represent the moiety $-CH_2-(CH_2)_n-Z-(CH_2)_m-$ as hereinbefore defined and the moiety $R_5.N.CX.R_6$ represents either pyrrolidonyl or piperidonyl.

When Z is other than $CH_2$, m is often 0 or 1 and n is often 0 or 1.

When Z represents NR, suitable arylcarbonyl groups include naphthylcarbonyl, phenylcarbonyl or benzylcarbonyl optionally substituted in the phenyl or naphthyl ring by one or two of alkyl, alkoxy or halogen.

Suitable examples of R when Z is NR include hydrogen, methyl, ethyl, n- and iso-propyl, n-, sec- and tert-butyl, benzyl, phenylcarbonyl or benzylcarbonyl optionally substituted in the phenyl ring by methyl, methoxy, chloro or bromo; furylcarbonyl, thienylcarbonyl, pyrrolylcarbonyl or indolylcarbonyl.

Preferably R is hydrogen, methyl, n-butyl, acetyl, benzyl, benzylcarbonyl, phenylcarbonyl or furylcarbonyl.

Most preferably R is methyl.

In a particular aspect of the invention, $R_5$ and $R_6$ together represent $C_3$-or $C_4$-polymethylene when A represents $>C=X$.

Suitable unsaturated, heterocyclic rings represented by the moiety $R_6.N.R_5$ include those having 5- or 6- ring atoms, favourably 6- ring atoms.

Suitable optional substituents for the ring atoms of the unsaturated, heterocyclic ring represented by $R_6.N.R_5$, include alkyl, hydroxyl, thiol, oxo or thioxo and also those substituents which jointly provide fused rings, for example a fused benzene ring or a further heterocyclic ring, including any heterocyclic ring mentioned above.

Favourably, when A represents a bond, the moiety $R_6.N.R_5$ represents a substituted or unsubstituted pyridonyl or substituted or unsubstituted thiopyridonyl group, especially a pyridonyl group. An especially favoured pyridonyl group is a 2-pyridon-1-yl group.

Suitably p represents 1. Suitably p represents zero. Preferably p represents zero.

Preferably $R_7$ and $R_8$ are independently selected from the class of hydrogen, alkyl, $C_{3-8}$ cycloalkyl, fluoroalkyl, alkylcarbonyl, alkoxycarbonyl, nitro, cyano or amino, providing that at least one of $R_7$ or $R_8$ is not hydrogen. In particular, $R_7$ and $R_8$ may each independently represent hydrogen, acetyl, nitro, cyano, amino, methyl, ethyl, isopropyl, cyclopentyl, trifluoromethyl or pentafluoroethyl, providing that at least one of $R_7$ or $R_8$ is not hydrogen. Preferably, $R_7$ and $R_8$ may each independently represent hydrogen, nitro, cyano, amino, methyl, ethyl, trifluoromethyl or pentafluoroethyl, providing that at least one of $R_7$ or $R_8$ is not hydrogen.

When one of $R_7$ and $R_8$ represents nitro, cyano or alkylcarbonyl, suitably $C_{1-3}$ alkylcarbonyl, the other may represent amino optionally substituted by one or two $C_{1-6}$ alkyl or by $C_{2-7}$ alkanoyl. In particular, when one of $R_7$ and $R_8$ is nitro, cyano or $C_{1-3}$ alkylcarbonyl, the other is amino, methylamino,dimethylamino or acetylamino. For example when one of $R_7$ and $R_8$ is nitro or cyano, the other may represent amino.

When one of $R_7$ and $R_8$ is nitro, cyano or alkylcarbonyl, $R_8$ may represent nitro, cyano or alkylcarbonyl.

When one of $R_7$ or $R_8$ represents hydrogen the other is preferably selected from the class of alkyl, $C_{3-8}$ cycloalkyl, fluoroalkyl, alkylcarbonyl, alkoxycarbonyl, nitro, cyano or amino, and in particular the other is selected from acetyl, nitro, cyano, amino, methyl, ethyl, isopropyl, cyclopentyl, trifluoromethyl or pentafluoroethyl.

Unless mentioned to the contrary, $R_7$ suitably represents hydrogen and $R_8$ represents any of the variables mentioned above in regard to $R_7$ or $R_8$.

Unless mentioned to the contrary, $R_8$ suitably represents hydrogen and $R_7$ represents any of the variables mentioned above in regard to $R_7$ or $R_8$.

In one preferred aspect $R_7$ represents hydrogen and $R_8$ represents nitro, cyano, amino, ethyl, trifluoromethyl or pentafluoroethyl, especially nitro, cyano or ethyl.

In one preferred aspect $R_8$ represents hydrogen and $R_7$ represents nitro, cyano, amino, ethyl, trifluoromethyl or pentafluoroethyl, especially cyano, ethyl, trifluoromethyl or pentafluoroethyl.

When used herein the term "halogen" refers to fluorine, chlorine, bromine and iodine; preferably chlorine.

Suitably alkyl groups, or alkyl groups forming part of other groups such as in the alkoxy group, are

$C_{1-12}$ alkyl groups especially $C_{1-6}$ alkyl groups e.g. methyl, ethyl, n-propyl, iso-propyl, n-butyl, isobutyl or tert-butyl groups.

Suitably alkenyl groups are $C_{2-12}$ groups especially $C_{2-6}$ alkenyl groups.

Suitable alkynyl groups are $C_{2-12}$ alkynyl groups especially $C_{2-6}$ alkynyl groups.

Suitable polymethylene groups include $C_3$, $C_4$, $C_5$, $C_6$ and $C_7$ polymethylene groups.

Suitable acyloxy groups are alkylcarbonyloxy groups wherein the 'alkyl' group is as defined above.

When used herein the term "fluoroalkyl" includes alkyl groups as defined above when substituted by one or more fluorine atoms, particular examples being trifluoromethyl and pentafluoroethyl.

Suitable pharmaceutically acceptable salts of the compounds of formula (I) include acid addition salts and salts of carboxy groups.

Examples of pharmaceutically acceptable acid addition salts of the compounds of formula (I) includes acid addition salts of optionally substituted amino groups, such as the hydrochloride and hydrobromide salts. Such a salifiable group may form part of an $R_5$ group. It will also be appreciated that when Y in the compound of formula (I) represents N, then the resulting pyridine moiety may yield acid addition salts, such as the hydrochloride or hydrobromide salts.

Examples of pharmaceutically acceptable salts of carboxy groups include metal salts, such as alkali metal salts, or optionally substituted ammonium salts.

Examples of esters of carboxy groups are pharmaceutically acceptable esters such as $C_{1-6}$ alkyl esters.

Examples of amides of carboxyl groups include pharmaceutically acceptable amides such as amides of formula $-CO.NR^sR^t$ wherein $R^s$ and $R^t$ each independently represent hydrogen or $C_{1-6}$ alkyl.

The compounds of formula (I) may also exist in the form of solvates, preferably hydrates, and the invention extends to such solvates.

The compounds of formula (I), may exist in the form of optical isomers. For example chirality is present in those compounds of formula (I) wherein $R_3$ is hydrogen, hydroxy, alkoxy or acyloxy and $R_4$ is hydrogen. Chirality may also arise in those compounds of formula (I) wherein $R_5$ and $R_6$ together represent a linking group $-A^1-A^2-$, as the said linking group may possess up to 4 chiral carbon atoms. The present invention extends to all optical isomers of the compounds of formula (I) whether in the form of single isomers or of mixtures thereof, such as racemates.

The compounds of formula (I) may also exist in geometrical isomeric forms all of which are encompassed by the present invention, including those wherein the $R_6.N.AR_5$ moiety and $R_3$ are disposed either mutually cis with respect to one another or, preferably, mutually trans with respect to one another.

Particular examples of compounds of formula (I) include the compounds prepared in the Examples hereinafter.

The present invention also provides a process for the preparation of a compound of formula (I) or, where appropriate a pharmaceutically acceptable salt thereof, or a pharmaceutically acceptable solvate thereof, which comprises;

i) for compounds of formula (I) wherein p represents zero and A represents >C=X, acylating a compound of formula (II):

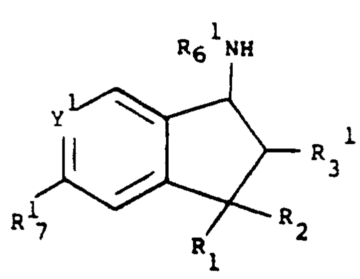

(II)

wherein, $Y^1$ represents Y or a group convertible to Y, $R_1$ and $R_2$ are as defined hereinbefore, $R_3{}^1$ is hydroxy, alkoxy or acyloxy, $R_6{}^1$ is hydrogen or alkyl and $R_7{}^1$ represents $R_7$ or a group or atom convertible to $R_7$,

a) with an acylating agent of formula (III):

$R_{10}$-CO-$L_1$ (III)

wherein $L_1$ is a leaving group, and $R_{10}$ is hydrogen, substituted or unsubstituted alkyl wherein the

substituents are as hereinbefore defined for $R_5$; or amino optionally substituted as hereinbefore defined for $R_5$; alkenyl; or optionally substituted aryl or heteroaryl as hereinbefore defined for $R_5$, or a group convertible to $R_5$ as hereinbefore defined, and thereafter, when $R_6$ is hydrogen and $R_{10'}$ is $X^1CH_2(CH_2)_z$, where z is 2 or 3; and $X^1$ is a leaving group, cyclising the resultant compound;

b) with a compound of formula (IV)

$X=C=N.R_{11}$ (IV)

wherein $R_{11}$ is hydrogen, alkyl, alkenyl, alkanoyl optionally substituted by up to three halo atoms, or phenyl optionally substituted by alkyl, alkoxy or halogen; and X is oxygen or sulphur, and thereafter when $R_{11}$ is hydrogen, optionally converting $R_{11}$; or

ii) for compounds of formula (I) wherein p represents zero and A represents >C=X and $R_5$ and $R_6$ together represent a linking chain of formula $-A^1-A^2-$ as defined above in relation to formula (I), by reacting a compound of formula (V):

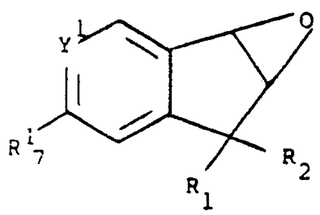

(V)

wherein $R_1$ and $R_2$ are as hereinbefore defined in relation to formula (I) and $R_7^1$ and $Y^1$ are as defined in relation to formula (II), with an activated form of a compound of formula (VIA):

$R_{13}NHCOR_{12}$ (VIA)

wherein $R_{12}$ and $R_{13}$ together represent a linking chain of formula $-A^1-A^2-$;

iii) for compounds of formula (I) wherein p represents zero and A represents a bond and $R_5$ and $R_6$ together with the nitrogen to which they are attached, form the above defined unsaturated heterocyclic ring, by reacting a compound of formula (V) as defined above with an activated form of a compound of formula (VIB):

$R_6'NHR_5'$ (VIB)

wherein $R_5'$ and $R_6'$ form an unsaturated heterocyclic ring as defined above in respect of the variables $R_5$ and $R_6$, or

(iv) for compounds of formula (I) wherein p represents 1, reacting a compound of formula (VII):

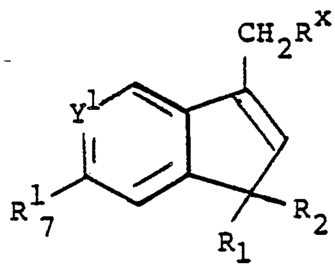

(VII)

wherein $R_1$ and $R_2$ are as defined in relation to formula (I), $Y^1$ represents Y or a group convertible to Y, $R_7^1$ represents $R_7$ or a group convertible to $R_7$ and $R^x$ represents a leaving group, with a compound of formula (VIII):

$R_6-NH-A-R_5$ (VIII)

or an activated form thereof, wherein $R_5$, $R_6$ and A are as defined in relation to formula (I):

and thereafter if required, carrying out one or more of the following optional steps:

(a) converting a compound of formula (I) into a further compound of formula (I);

(b) converting $Y^1$ to Y;

(c) converting $R_7^1$ to $R_7$;

(d) forming a pharmaceutically acceptable salt of the compound of formula (I);

(e) forming a pharmaceutically acceptable solvate of the compound of formula (I) or a pharmaceutically acceptable salt thereof.

In the process variant i) a) acylation of a compound of formula (II) with an acylating agent of formula (III), the leaving group $L_1$ is a group that is displaceable by a primary or secondary amino nucleophile. Examples of such a group include $C_{1-4}$ alkanoyloxy, and halogen, such as chloro and bromo. When the leaving group $L_1$ is either of these examples, the acylating agent of formula (III) is either an acid anhydride or an acid halide. When it is an acid anhydride, it may be a mixed or simple anhydride. If it is a mixed anhydride, it may be prepared in situ from a carboxylic acid and an acid halide, although this is less preferred than using the halide itself. When $L_1$ is hydroxy, conventional coupling methods using dicyclohexylcarbodiimide are suitable.

In process variant i) a), when $R_5$ in the desired compound of formula (I) is an optionally substituted amino-substituted alkyl group as hereinbefore defined, it is preferred that $R_{10}$ is a group convertible to the $R_5$ substituted alkyl group as hereinbefore defined, in particular that it is $C_{1-6}$ alkyl substituted by halo, especially bromo. The $R_{10}$ halo substituent in the resultant compound of process variant i) a) may be converted to an $R_5$ substituent which is amino optionally substituted as hereinbefore defined by a conventional amination reaction with ammonia or a corresponding alkyl- or dialkylamine.

Less favourably, $R_{10}$ may be alkyl substituted by protected amino, protected alkylamino or amino substituted by two independent alkyl groups, it being necessary to protect the $R_9$ amino function in process variant i) a).

When the acylating agent of formula (III) is an acid anhydride, the acylation of the compound of formula (II) may be carried out in the presence of an acid acceptor, such as sodium acetate, optionally using the anhydride as the solvent.

When the acylating agent of formula (III) is an acid halide, the acylation of the compound of formula (II) is, preferably, carried out in a non-aqueous medium, such as dichloromethane, in the presence of an acid acceptor, such as triethylamine, trimethylamine, pyridine, picoline or calcium, potassium or sodium carbonate.

When $R_3{}^1$ in a compound of formula (II) is hydroxy, there is a risk of a side-reaction between the hydroxy group and the acylating agent of formula (III). However, the reaction may be carried out under controlled conditions such that only the amine, $R_6{}^1NH$-is acylated, for example, by using a $C_{2-9}$ acyloxy group as the leaving group $L_1$, in the acylating agent of formula (III) in the manner as previously described for an acid anhydride, and/or effecting the reaction at relatively low temperature, e.g. at below 10° C.

Alternatively $R_3{}^1$ may be acyloxy in a compound of formula (II), although less preferably if $R_3$ in the resultant compound of formula (I) is to be hydroxy, and, after reaction with the acylating agent of formula (III), be converted into hydroxy, as described hereinafter.

When $R_{10}$ is $X^1(CH_2)_z$ wherein the variables $X^1$ and z are as hereinbefore defined, the leaving group $X^1$ is a group that is displaceable by a secondary amino nucleophile adjacent to a carbonyl function. A preferred example is chloro.

The cyclisation reaction in the reaction wherein $R_{10}$ represents $X^1(CH_2)_z$ as defined above is preferably carried out in an inert solvent such as dimethylformamide.

In process variant i) b), when $R_{11}$ in a compound of formula (IV) is alkyl, alkanoyl optionally substituted as hereinbefore defined, or phenyl optionally substituted as hereinbefore defined, the reaction between the compounds of formulae (II) and (IV) is, preferably, carried out in a solvent, such as methylene chloride, at below room temperature, in particular below 10° C.

When $R_{11}$ is hydrogen, the reaction between the compounds of formulae (II) and (IV) is, preferably, carried out using a corresponding alkali metal cyanate or thiocyanate, for example that of sodium or potassium, in an optionally methanolic aqueous medium acidified with a mineral acid, such as dilute hydrochloride acid. A slightly elevated temperature such as 50 to 90° C is apt.

In process variant ii), a suitable activated form of a compound of formula (VIA) is an ionic form. Thus in the reaction between a compound of formula (V) and a compound of formula (VIA), it is preferred that the reaction is carried out under basic conditions so as to facilitate the formation of the anion of the compound of formula (VI), for example, in the prsence of an alkali metal base such as potassium t-butoxide or sodium hydride.

The reaction between the compounds of formula (V) and (VIA) may be carried out in any suitable aprotic solvent at a temperature that provides a convenient rate of formation of the compound of formula (I), such as at ambient temperature or at an elevated temperature,for example 40° C.

Conveniently, the compound of formula (VIA) may itself be used as the solvent for the reaction between compounds of formulae (V) and (VIA).

In process variant iii), a suitable activated form of a compound of formula (VIB) is an ionic form. Thus in the reaction between a compound of formula (V) and a compound of formula (VIB), it is preferred that the reaction is carried out under basic conditions so as to facilitate the formation of the anion of the compound of formula (VIB), for example, in the presence of an alkali metal base such as potassium t-butoxide or sodium hydride.

The reaction between the compounds of formulae (V) and (VIB) may be carried out in any suitable aprotic solvent, for example dimethylsulphoxide, at a temperature that provides a convenient rate of formation of the compound of formula (I), such as at ambient temperature or at an elevated temperature, but conveniently at ambient temperature.

In process variant iv) a suitable activated form of a compound of formula (VIII) is an ionic form. Thus in the reaction between a compound of formula (VII) and a compound of formula (VIII), it is preferred that the reaction is carried out under basic conditions so as to facilitate the formation of the anion of the compound of formula (VIII), for example, in the presence of an alkali metal base such as potassium t-butoxide or sodium hydride.

The reaction between the compounds of formulae (VII) and (VIII) may be carried out in any suitable aprotic solvent, for example tetrahydrofuran, at a temperature that provides a convenient rate of formation of the compound of formula (I), such as at ambient temperature or at an elevated temperature.

Suitably, $R^x$ in the compound of formula (VII) represents a bromine atom.

Suitable conversions of a compound of formula (I) to a further compound of formula (I) include:

(i) converting $R_3$ in the resulting compound of formula (I) into another $R_3$;

(ii) converting a compound of formula (I) wherein $R_3$ and $R_4$ represent hydroxy and hydrogen respectively to give another compound of formula (I), wherein $R_3$ and $R_4$ together represent a bond;

(iii) reducing any compound of formula (I) wherein $R_3$ and $R_4$ together represent a bond; to give another compound of formula (I), wherein $R_3$ and $R_4$ each represent hydrogen;

(iv) thiating a compound of formula (I) to convert any -CO- group in the moiety of formula $R_6$.N.A.$R_5$ into a -CS- group; or

(v) when $R_3$ is other than hydrogen, interconverting the cis and trans mutual configuration of the variables $R_3$ and $R_4$.

The reaction of the compounds of formulae (II) with (III) or (IV) results in a compound of formula (I) wherein $R_3$ is hydroxy, alkoxy or acyloxy, whereas the reaction of the compounds of formulae (V) and (VIA) and (V) with (VIB) results in a compound of formula (I) wherein $R_3$ is hydroxy. Examples of an optional conversion of $R_3$ in a compound of formula (I) into another $R_3$ are generally known in the art. For example, when $R_3$ is hydroxy, it may be alkylated using an alkyl iodide in an inert solvent, such as toluene, in the presence of a base, such as sodium hydride or potassium hydroxide, or it may be acylated using a carboxylic acid chloride or an appropriate anhydride in a non-hydroxylic solvent, such as toluene or dichloromethane, in the presence of an acid acceptor such as triethylamine. When $R_3$ is alkoxy it may be converted into a hydroxy group by any conventional dealkylation method for example by treatment with trimethylsilyliodide in an aprotic solvent. In addition, when $R_3$ is acyloxy it may be converted into hydroxy by conventional hydrolysis using, for example, dilute mineral acid.

The optional conversion of a compound of formula (I), wherein $R_3$ and $R_4$ are hydroxy and hydrogen respectively, into another compound of formula (I), wherein $R_3$ and $R_4$ together are a bond, may be carried out by dehydration under conventional dehydration conditions, for example, by using a dehydrating agent, such as sodium hydride, in inert solvent, such as dry tetrahydrofuran, at reflux temperature; alternatively the hydroxy group represented by $R_3$ may be converted into a leaving group such as a mesyloxy or tosyloxy group and the resulting compound treated with a base such as sodium hydride to provide the compound of formula I) wherein $R_3$ and $R_4$ together represent a bond.

The reduction of a compound of formula (I), wherein $R_3$ and $R_4$ together are a bond, into another compound of formula (I), wherein $R_3$ and $R_4$ are each hydrogen, may be carried out by hydrogenation using a catalyst of palladium on charcoal.

The thiation of the $R_6$.N.A.$R_5$ moiety in a compound of formula (I) to give another compound of formula (I), is, preferably, carried out with conventional thiation agents, such as hydrogen sulphide, phosphorus pentasulphide and Lawesson's reagent (p-methoxyphenylthiophosphine sulphide dimer). The use of hydrogen sulphide and phosphorus pentasulphide may lead to side-reactions and, therefore, the use of Lawesson's reagent is preferred. The thiation reaction conditions are conventional for the thiation agent employed. For example, the use of hydrogen sulphide is, preferably, acid catalysed by, for example, hydrogen chloride in a polar solvent, such as acetic acid or ethanol. The preferred use of Lawesson's reagent is, preferably, carried out under reflux in a dry solvent, such as toluene or methylene chloride.

The interconversion of the cis and trans configuration of the variables $R_3$ and $R_4$ is generally carried out by changing the configuration of variable $R_3$, especially when $R_3$ represents hydroxyl, by means of any convenient conventional procedure.

The required conversion of $Y^1$ to Y or $R_7{}^1$ to $R_7$ may be carried out using the appropriate conventional chemical procedure.

The optional formation of a pharmaceutically acceptable salt, when the resulting compound of formula (I) contains a salifiable group, may be carried out conventionally. Similarly, pharmaceutically acceptable solvates, for example hydrates, may be prepared using any convenient conventional procedure.

A compound of formula (II) may be prepared by reacting a compound of formula (V), as defined hereinbefore, with a compound of formula (IX):

$R_6{}^1NH_2$ (IX)

wherein $R_6{}^1$ is as defined hereinbefore; and optionally converting $R_3{}^1$ hydroxyl in the resulting compound of formula (II) into another $R_3{}^1$.

The reaction between the compounds of formula (V) and (IX) is normally carried out in a solvent, such as a $C_{1-4}$ alcohol, in particular methanol, ethanol or propanol at an ambient or an elevated temperature, for example 12 to 100° C. The reaction proceeds particularly smoothly if carried out in ethanol under reflux.

A compound of formula (II) may also be prepared by reacting a compound of the hereinbefore formula (V) with an ionic azide, preferably an alkali metal azide such as sodium azide, and thereafter reducing the resulting azido product, with for example sodium borohydride.

The reaction between the compound of formula (V) and the ionic azide may be carried out in any suitable solvent, for example aqueous dioxan, conveniently at ambient temperature. The reduction of the resulting azido compound may be carried out using the appropriate conventional reducing conditions, for example in methanolic tetrahydrofuran conveniently at reflux.

The resulting compound of formula (II) may be recovered from the reaction mixture by removal of the solvent, for example, by evaporation under reduced pressure. Any epoxide impurity may be removed conventionally, for example by chromatography.

The optional conversion of the hydroxy group for $R_3{}^1$ in the resulting compound of formula (II) into a $C_{1-6}$ alkoxy or $C_{1-7}$ acyloxy group may be carried out as described hereinbefore in relation to the corresponding conversion of $R_3$ in a compound of formula (I).

A compound of formula (V) may be prepared by reaction of a compound of formula (X):

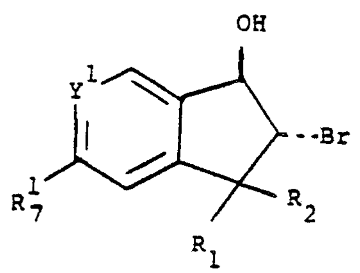

(X)

wherein $R_1$, $R_2$, $R_7{}^1$ and $Y^1$ are as hereinbefore defined, the bromine atom being trans to the hydroxy group, with a base, such as potassium hydroxide or potassium tert butoxide, in a non-aqueous solvent, such as ether or dimethylsulphoxide or in an aqueous solvent such as aqueous dioxan.

Suitably, the compound of formula (V) is not isolated from the last abovementioned reaction when carried out in a non-aqueous solvent and is reacted in situ with either a compound of formula (VIA) or (VIB) to provide the appropriate compound of formula (I).

A favoured base is potassium tert-butoxide.

Accordingly, in one preferred aspect the present invention provides a process for the preparation of a compound of formula (I) or, where appropriate a pharmaceutically acceptable salt thereof, or a pharmaceutically acceptable solvate thereof which comprises reacting a compound of formula (X), as defined above, with a base to provide a compound of the above defined formula (V) and thereafter reacting the compound of formula (V) in situ with either a compound of formula (VIA) or (VIB) as respectively described above in reaction ii) or reaction iii).

A compound of formula (X) may be prepared by reaction of a compound of formula (XI):

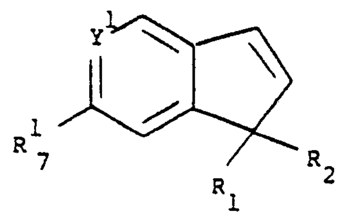

(XI)

wherein $R_1$, $R_2$, $R_7{}^1$ and $Y^1$ are as defined hereinbefore, with N-bromosuccinimide in a solvent, such as aqueous dimethyl sulphoxide.

A compound of formula (V) may also be prepared directly from a compound of the hereinbefore defined (XI), by reacting the compound of formula (XI) with a per-acid, preferably a perbenzoic acid, and in particular with meta-chloroperbenzoic acid.

The reaction between the compound of formula (XI) and the per-acid may be carried out in any suitable inert solvent such as dichloromethane at any suitable temperature, conveniently at ambient temperature.

A compound of formula (XI) may be prepared by dehydrating a compound of formula (XII):

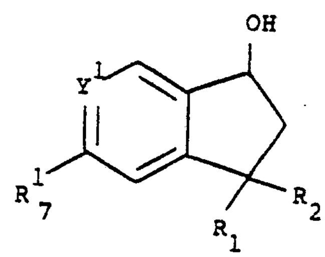

(XII)

wherein $R_1$, $R_2$, $R_7{}^1$ and $Y^1$ are as hereinbefore defined.

The dehydration of a compound of formula (XII) may conveniently be carried out by heating a compound of formula (XII) in any suitable solvent, for example benzene or toluene, preferably in the presence of a catalyst such as p-toluenesulphonic acid. Suitably the reaction is carried out at the reflux temperature of the solvent. Preferably the water produced in the reaction is removed from the reaction mixture, for example by carrying the reaction out in a Dean-Stark apparatus at the reflux temperature of the chosen solvent, such as benzene.

A compound of formula (XII) may be prepared by reducing a compound of formula (XIII):

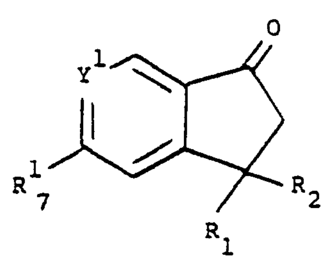

(XIII)

wherein $R_1$, $R_2$, $R_7$ and Y are as hereinbefore defined.

The reduction of a compound of formula (XIII) may be carried out using any suitable reducing agent, such as a complex metal hydride reagent for example potassium borohydride, in any suitable solvent, such as a $C_{1-4}$ alkanol, for example methanol.

A compound of formula (VII) may be prepared by reacting a compound of formula (XIV)

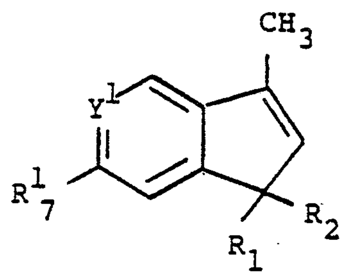

(XIV)

wherein $R_1$, $R_2$, $R_7{}^1$ and $Y^1$ are as defined in relation to formula (VII), with a reagent capable of converting $-CH_3$ to $-CH_2R^x$.

When $R^x$ is bromine, in the compound of formula (VII), a suitable reagent is N-bromosuccinimide.

The reaction conditions for the reaction between the compound of formula (XIV) and the reagent will depend upon the particular reagent chosen, for example when the reagent is N-bromosuccinimide the reaction may be carried out in any suitable inert solvent, such as carbon tetrachloride, at an ambient to elevated temperature, conveniently the reflux temperature of the solvent, and preferably in the presence of ultraviolet light and preferably in the presence of an appropriate catalyst such as azobisisobutyronitrile.

A compound of formula (XIV) may be prepared by isomerising a compound of formula (XV):

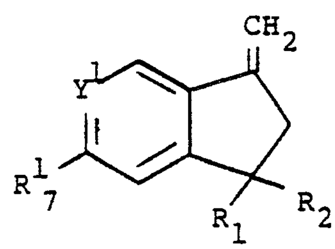

(XV)

wherein $R_1$, $R_2$, $R_7{}^1$ and $Y^1$ are as hereinbefore defined.

The isomerisation of the compound of formula (XV) may suitably be carried out by treating the compound of formula (XV) with an acid, for example trifluoroacetic acid, in any suitable inert solvent, such as dichloromethane, conveniently at ambient temperature.

A compound of formula (XV) may be prepared by the methyleneation of a compound of the above defined formula (XIII).

The methyleneation of the compound of formula (XIII) is suitably carried out using an appropriate Wittig reagent such as methylene triphenylphosphorane ($Ph_3P = CH_2$).

Reaction conditions for the methyleneation of the compound of formula (XIII) will depend upon the specific methyleneation procedure chosen. Thus the Wittig methyleneation of the compound of formula (XIII) may be carried out in any suitable aprotic solvent, such as tetrahydrofuran, at a temperature providing a suitable rate of formation of the compound of formula (XV), for example at ambient temperature.

Suitably the Wittig reagent is prepared in-situ, for example methylene triphenylphosphorane may conveniently be prepared from methyltriphenylphosphonium bromide and potassium t-butoxide at low to ambient temperature for example at -78° C.

A favoured alternative process for preparing the compounds of formula (XI) or (XIV), wherein $Y^1$ represents N or $N^+-O^-$, comprises dehydrobrominating a compound of formula (XVI):

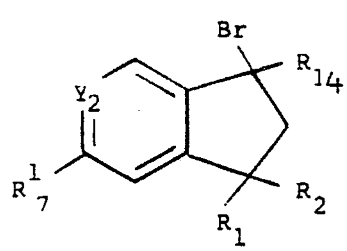

(XVI)

wherein $R_1$, $R_2$ and $R_7{}^1$ are as defined in relation to formula (XI), $Y_2$ represents N or $N^+$-$O^-$ and $R_{14}$ represents hydrogen, for preparing compounds of formula (XI), or methyl for preparing compounds of formula (XIV).

The dehydrobromination of a compound of formula (XVI) may be carried out under conventional dehydrobrominating conditions, generallly such conditions comprise treating (XVI) with a base, for example sodium methoxide, in any suitable solvent, such as methanol, conveniently at an elevated temperature.

A compound of formula (XVI) may suitably be prepared by brominating a compound of formula (XVII):

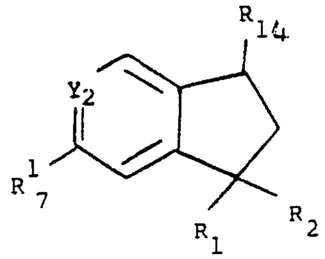

(XVII)

wherein $R_1$, $R_2$, $R_7{}^1$, $R_{14}$ and $Y_2$ are as defined in relation to formula (XVI).

A suitable brominating agent for brominating a compound of formula (XVII) is N-bromosuccinimide. Suitable brominating conditions are those discussed above for the bromination of compounds of formula (XIV).

The compounds of formula (XIII) are either known compounds or they may be prepared using methods analogous to those used to prepare known compounds, for example those disclosed in J. Amer. Chem. Soc. 1961, 1299 and Org. Prep. Proced. Int., 1978, 10(3), 123.

As mentioned previously, some of the compounds of formula (I) may exist in optically active forms, and the processes of the present invention produce mixtures of such forms. The individual enantiomers may be resolved by conventional methods.

It is preferred that the compounds of formula (I) are isolated in substantially pure form.

Esters and amides of carboxyl groups are prepared by the appropriate conventional procedure.

Salts and/or solvates of the compounds of formula (I) may be prepared by the appropriate conventional procedure.

The intermediates of formulae (II), (V), (VII), (X), (XI), (XII), (XIV), (XV) and (XVI) are believed to be novel and accordingly they form part of the present invention.

The intermediates of formulae (III), (IV), (VIA), (VIB), (VIII) or (IX) are known and may be prepared using conventional procedures, for example the procedures or analogous procedures to those disclosed in Advanced Organic Chemistry, 3rd Edition, (1985), Published by John Wiley and Sons.

The intermediates of formula (XVII) are known or they may be prepared using analogous methods to those used to prepare known compounds for example the methods disclosed in J. Org. Chem. 1981, 46, 2179-2182 or J. Org. Chem. 1982, 47, 895-897.

The compounds of formula (I), the pharmaceutically acceptable salts thereof or the pharmaceutically acceptable solvates thereof, have been found to have bronchodilator activity and/or blood-pressure lowering activity. They are therefore useful in the treatment of respiratory tract disorders, such as reversible airways obstruction, diverticular disease and asthma and also hypertension. They may also be of potential use in the treatment of other disorders hereinbefore described.

The present invention accordingly provides a pharmaceutical composition which comprises a compound of formula (I), or a pharmaceutically acceptable salt thereof or a pharmaceutically acceptable solvate thereof, and a pharmaceutically acceptable carrier.

The compositions are preferably adapted for oral administration. However, they may be adapted for other modes of administration, for example in the form of a spray, aerosol or other conventional method for inhalation, for treating respiratory tract disorders; or parenteral administration for patients suffering from heart failure. Other alternative modes of administration include sublingual or transdermal administration.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or

polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Compositions of this invention may also suitably be presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns. Where appropriate, small amounts of other anti-asthmatics and bronchodilators, for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included.

The compositions may contain from 0.1% to 99% by weight, preferably from 10-60% by weight, of the active material, depending on the method of administration.

The present invention further provides a method of treatment of respiratory tract disorders or hypertension in mammals including man, which method comprises administering an effective, non-toxic amount of a compound of formula (I), or a pharmaceutically acceptable salt thereof or a pharmaceutically acceptable solvate thereof to the mammal in need thereof.

The present invention also provides a method of treatment of disorders associated with smooth muscle contraction of the gastro-intestinal tract and the abovementioned cardiovascular disorders in mammals including man, which method comprises administering an effective, non-toxic amount of a compound of formula (I), or a pharmacetically acceptable salt thereof or a pharmaceutically acceptable solvate thereof to the mammal in need thereof.

An effective amount will depend on the relative efficacy of the compounds of the present invention, the severity of the respiratory tract disorder or hypertension being treated and the weight of the sufferer. However, a unit dose form of a composition of the invention may contain from 0.01 to 100mg of a compound of the invention (0.01 to 10mg via inhalation) and more usually from 0.1 to 50mg, for example 0.5 to 25mg such as 1, 2, 5, 10, 15 or 20mg. Such compositions may be administered from 1 to 6 times a day, more usually from 2 to 4 times a day, in a manner such that the daily dose is from 0.02 to 200mg for a 70 kg human adult and more particularly from 0.05to 100mg.

Suitable dosages for the treatment of disorders associated with smooth muscle contraction of the gastro intestinal tract and the abovementioned cardiovascular disorders are those described for the treatment of respiratory tract disorders.

The present invention further provides a compound of formula (I) or a pharmaceutically acceptable salt thereof, or a pharmaceutically acceptable solvate thereof, for use in the treatment of respiratory tract

disorders or hypertension or disorders associated with smooth muscle contraction of the gastro intestinal tract or the abovementioned cardiovascular disorders.

The present invention further provides the use of a compound of formula (I), or a pharmaceutically acceptable salt thereof, or a pharmaceutically acceptable solvate thereof for the manufacture of a medicament for the treatment of respiratory tract disorders or hypertension or disorders associated with smooth muscle contraction of the gastro intestinal tract or the abovementioned cardiovascular disorders.

The following Procedures relate to the preparation of intermediates and the following Examples relate to the preparation of compounds of formula (I).

Examples 1 and 2

Cis and Trans 1,1-Dimethyl-5-nitro-3-(2-oxopyrrolidin-1-yl)indan-2-ol

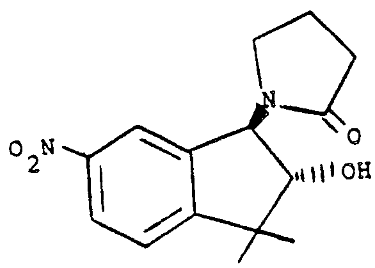

Example 1

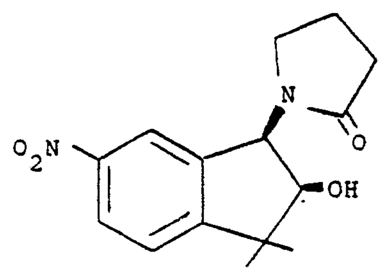

Example 2

To a solution of 1,1-dimethyl-2,3-epoxy-5-nitroindane (0.54g, 2.6mmol) in 2-pyrrolidinone (6ml) was added potassium t-butoxide (0.41g, 3.7mmol). The solution was stirred for 3.5h then poured into water (100ml) and the solution extracted with chloroform (60ml). The chloroform phase was washed with water (3 x 50ml), dried ($MgSO_4$), and concentrated to a yellow oil. Chromatography on silica gel (3:2 ethyl acetate/hexane to ethyl acetate; gradient elution) provided first cis-1,1-dimethyl-5-nitro-3-(2-oxopyrrolidin-1-yl) indan-2-ol (0.18g, 24%), m.p. 144-145 °C followed by trans - 1,1-dimethyl-5-nitro-3-(2-oxopyrrolidin-1-yl)-indan-2-ol (0.18g, 24%), m.p. 222-223 °C ($CHCl_3$).

Trans isomer (Example 1)

$^1$H n.m.r. (DMSO-$d_6$):

δ 1.1 (s, 3H); 1.4 (s, 3H); 2.45 (m, 2H); 2.5 (m, 2H); 3.15 (m, 1H); 3.4 (m, 1H); 4.05 (d.d., J = 9.5, 5.5 $H_z$, 1H); 5.3 (d, J = 9.5 $H_z$, 1H); 5.65 (d, J = 5.5 $H_z$, 1H); 7.65 (d, J = 8 $H_z$, 1H); 7.75 (d, J= 2 $H_z$); 8.2 (dd, J= 8, 2$H_z$, 1H).

| Anal: | | | |
|---|---|---|---|
| Found,<br>Requires | C, 61.97;<br>C, 62.05, | H, 6.07;<br>H, 6.26; | N, 9.77%.<br>N, 9.65% for $C_{15}H_{18}N_2O_4$. |

Cis isomer (Example 2)

$^1$H n.m.r. ($CDCl_3$):

δ 1.25 (s, 3H); 1.4 (s, 3H); 2.1 (m, 2H); 2.55 (t, J = 8 Hz, 2H); 3.0 (d, J = 6.5 Hz, 1H); 3.5 (m, 2H); 4.35 (t, J = 5.5 Hz, 1H); 5.6 (d, J = 5.5 Hz, 1H); 7.4 (d, J = 8.5 Hz, 1H); 7.95 (d, J =1.5 Hz, 1H); 8.2 (d.d., J = 8.5, 1.5 Hz, 1H).

| Anal: | | | |
|---|---|---|---|
| Found | C, 61.85; | H, 6.23; | N, 9.61%. |
| Requires | C, 62.05; | H, 6.26; | N, 9.65% for $C_{15}H_{18}N_2O_4$. |

Examples 3 and 4

Trans 1,1-Dimethyl-5-nitro-3-(2-oxopiperidin-1-yl) indan-2-ol and 1,1-dimethyl-5-nitro-3-(2-oxopiperidin-1-yl)-ind-2-ene

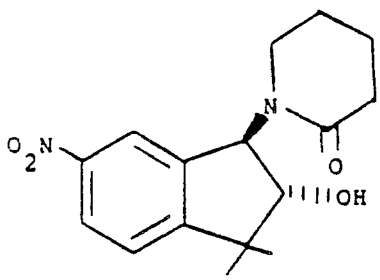

(Example 3)

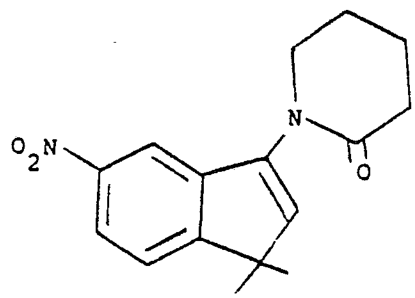

(Example 4)

1,1-Dimethyl-2,3-epoxy-5-nitroindane (0.57g, 2.8mmol) was added to a solution of potassium t-butoxide (0.36g, 3.2mmol) in 2-piperidone (5ml). The mixture was heated at 40°C for 3h, allowed to cool and hydrochloric acid solution (30ml, 0.5M) was added. Extraction with ether provided the crude products after drying ($MgSO_4$) and evaporation of the solvent. Purification by chromatography on silica gel eluting with ethyl acetate provided first starting epoxide (50mg) followed by (1,1-dimethyl-5-nitro-3-(2-oxopiperidin-1-yl) indene (40mg, 5%) m.p. 113-114°C and finally trans 1,1-dimethyl-5-nitro-3-(2-oxopiperidin-1-yl)indan-2-ol, m.p. 201°C.

Indanol:- (Example 3)

$^1$H n.m.r. ($CDCl_3$):

δ: 1.2 (s, 3H); 1.4 (s, 3H); 1.9 (m, 4H); 2.6 (m, 2H); 3.15 (t, J = 4.5 Hz, 2H); 3.5 (d, J = 7 Hz, 1H); 4.1 (d.d., J = 9.5, 7 Hz, 1H); 6.15 (d, J = 9.5 Hz); 7.3 (d, J = 8 Hz, 1H); 7.85 (d, J = 1 Hz, 1H); 8.15 (d.d., J = 8, 1 Hz, 1H).

Indene:- (Example 4)

$^1$H n.m.r. ($CDCl_3$):

δ 1.4 (s, 6H); 2.0 (m, 4H); 2.6 (m, 2H); 3.65 (m, 2H); 6.3 (s, 1H); 7.4 (d, J = 8 Hz, 1H); 7.9 (d, J = 2 Hz, 1H); 8.1 (d.d., J = 8, 2 Hz, 1H).

Example 5

Trans-1,1-dimethyl-5-nitro-3-(2-pyridon-1-yl)indan-2-ol

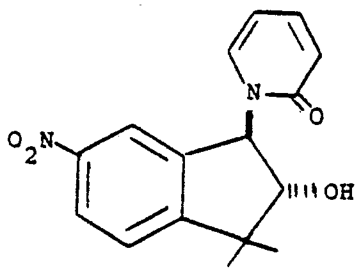

To a solution of 2-pyridone (6.53g, 68.7,mmol) and potassium t-butoxide (7.26g, 64.8mmol) in DMSO (30ml) was added 1,1-dimethyl-2,3-epoxy-5-nitroindane (2.83g, 13.8mmol) and the solution stirred for 72 hours. The reaction was quenched with 2N HCl (50ml) and extracted with ethyl acetate. The organics were washed with brine (3x75ml), dried ($MgSO_4$) and concentrated under reduced pressure. Chromatography of the residue on silica (gradient elution 2:1 ethyl acetate/hexane to ethyl acetate) yielded trans-1,1-dimethyl-5-nitroindan-2,3-diol (0.87g, 28%) and then trans-1,1-dimethyl-5-nitro-3-(2-pyridon-1-yl)indan-2-ol (1.76g, 42%) m.p.198- 199 °C.

$^1$Hnmr (DMSO-$d_6$):

δ 1.13 (s,3H); 1.4 (s,3H);4.4 (br, 1H); 5.75 (d, J=6 Hz, 1H); 6.1 (br, 1H); 6.3 (m,1H); 6.5 (d, J=9 Hz, 1 H); 7.5 (m,2H): 7.6 (m, 2H); 8.2 (dd, J=8 and 2 Hz, 1H).

| Anal: | | | | |
|---|---|---|---|---|
| $C_{16}H_{16}N_2O_4$ | requires :<br>found: | C,63.98;<br>C,63.35; | H,5.38;<br>H,5.48; | N,9.33<br>N,8.99% |

Example 6

1,1-Dimethyl-5-nitro-3-(2-pyridon-1-yl)ind-2-ene

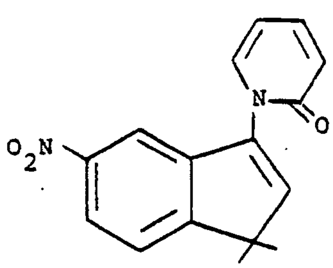

Methanesulphonyl chloride (0.11ml, 1.25mmol) was added to a solution of trans-1,1-dimethyl-5-nitro-3-(2-pyridon-1-yl) indan-2-ol (0.34g, 1.13mmol and triethylamine (0.17ml, 1.25mmol) in THF (15ml). The solution was stirred overnight, potassium t-butoxide (0.33g, 2.95mmol) added and the solution stirred for a further ½ hour, after which time the reaction was quenched with brine and extracted with ethyl acetate. The organics were separated, dried ($MgSO_4$) and concentrated to a brown solid. Chromatography on silica (ethyl

acetate) yielded 1,1-dimethyl-5-nitro-3-(2-pyridon-1-yl)ind-2-ene (0.28g, 88%) as a yellow solid, m.p 159-160°C.

$^1$Hnmr (DMSO-$d_6$):

δ 1.4 (s, 6H), 6.35 (dt,J=6.5 and 1.5Hz, 1H), 6.55 (d,J=9 Hz, 1 H), 6.85 (s, 1H), 7.15 (m, 3H),7.8 (d,J=8 Hz, 1 H), 8.2 (dd,J=8 and 2Hz, 1H).

| Anal: | | | | |
|---|---|---|---|---|
| $C_{16}H_{14}N_2O_3$ | requires<br>found | C, 68.06;<br>C, 68.25; | H,5.01;<br>H,4.94; | N 9.92<br>N9.84% |

Example 7

Trans-5-cyano-1,1-dimethyl-3-(2-pyridon-1-yl)-indan-2-ol

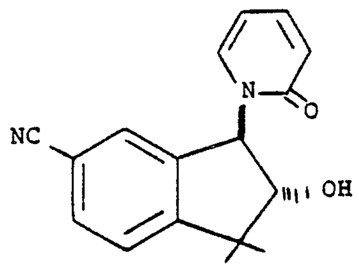

A solution of trans-5-amino-1,1-dimethyl-3-(2-pyridon-1-yl) indan-2-ol (0.92g, 3.41mmol) in ethanol (5ml) was added to conc.HCl (0.87ml) in water (10ml) at 0°C. To the cold mixture was added, dropwise, a solution of sodium nitrite (0.26g, 3.77mmol) in water (3ml) until an immediate positive reaction with starch-iodide paper was obtained. The reaction mixture was stirred for a further 10 minutes then added dropwise to a solution of potassium cyanide (2.07g, 30.1mmol) and cuprous cyanide (2.86g, 31.2mmol) in water (10ml) at 90°C at such a rate that the temperature did not fall below 70°C. On completion of addition heating was continued for a further 20 minutes after which time the reaction was allowed to cool and extracted with ethyl acetate. The organics were dried ($MgSO_4$) and concentrated in vacuo. Purification by chromatography on silica (ethyl acetate) yielded trans-5-cyano-1,1-dimethyl-3-(2-pyridon-1-yl)indan-2-ol (0.64g, 67%), m.p. 229-239°C (ethyl acetate).

$^1$Hnmr (DMSO-$d_6$):

δ 1.1 (s, 3H); 1.4 (s, 3H); 4.3 (br, 1H): 5.7 (d,J=6 Hz, 1H); 6.1 (br, 1H) 6.25 (dt, J=6.5 and 1 Hz, 1H): 6.45 (d,J=9 Hz, 1H); 7.2 (brs, 1 H); 7.5 (m, 3H); 7.7 (d,J=8 Hz, 1H).

Example 8

5-Cyano-1,1-dimethyl-3-(2-pyridon-1-yl)ind-2-ene

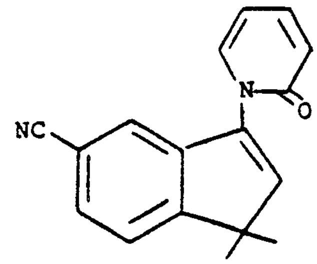

Methanesulphonyl chloride (0.1ml, 1.26mmol) was added to a solution of trans-5-cyano-1,1-dimethyl-3-(2-pyridon-1-yl) indan-2-ol (0.32g, 1.14mmol) and triethylamine (0.17ml, 1.26mmol) in THF (15ml), and the solution stirred for 5 hours whereupon potassium t-butoxide (0.282g, 2.51mmol) was added and the mixture stirred for a further hour. The reaction was quenched with brine, extracted with ethyl acetate, the organics dried ($MgSO_4$) and concentrated to a cream solid. Chromatography on silica (2:1 ethyl acetate/hexane) yielded 5-cyano-1,1-dimethyl-3-(2-pyridon-1-yl) ind-2-ene (0.26g, 87%), m.p. 140-141 °C

$^1$Hnmr ($CDCl_3$):

δ 1.45 (s, 6H); 6.25 (dt,J=7 and 1 Hz, 1H); 6.5 (s, 1H); 6.7 (d,J=8.5 Hz, 1H); 7.3 (dd, J=5.5 and 0.5Hz, 1H); 7.4 (m, 3H); 7.55 (dd,J=7.5 Hz and 1.5 Hz, 1H).

Example 9

Trans-5-amino-1,1-dimethyl-3-(2-pyridon-1-yl)indan-2-ol

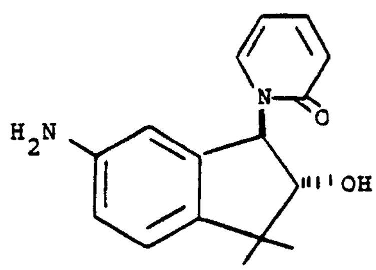

A mixture of trans-1,1-dimethyl-5-nitro-3-(2-pyridon-1-yl) indan-2-ol (1.02g, 3.4mmol) and 10% palladium on charcoal (0.21g) in methanol was hydrogenated until the required uptake had been achieved. The reaction mixture was filtered and concentrated to yield trans-5-amino-1,1-dimethyl-3-(2-pyridon-1-yl)indan-2-ol (0.92g, 100%) m.p. 143-144 °C.

$^1$Hnmr (DMSO-$d_6$/$CDCl_3$):

δ 1.1 (s, 3H); 1.35 (s, 3H); 3.3 (br, 1H); 4.0 (d,J=8.5Hz,1H); 4.05 (br, 1H); 4.9 (br, 1H); 6.2 (m, 2H); 6.3 (d,J=8.5Hz, 1H); 6.6 (m, 2H); 7.0 (d,J=7 Hz); 7.2 (dd, J=7 and 2 Hz, 1H); 7.35 (m,1H).

Example 10

Trans-1-N-(4-fluorobenzamido)-6-nitroindan-2-ol

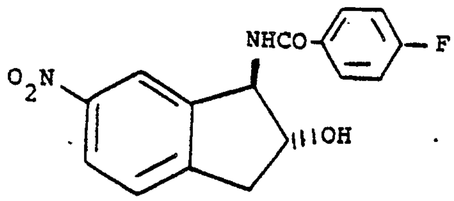

To a solution of 1-amino-6-nitroindan-2-ol (0.23g, 1.19mmol) in pyridine (10ml) was added 4-fluorobenzoyl chloride (0.16ml, 1.30mmol). The solution was stirred for 4.5 hours, poured into 4N HCl solution (100ml) and extracted with ethyl acetate. The organics were separated, dried ($MgSO_4$) and concentrated to a dark oil which was chromatographed on silica (ethyl acetate/5% ethanol) to give the product as a light brown solid. Further purification was effected by dissolving the solid in hot ethanol and heating with decolourizing charcoal (ca.3g) for 3 hours. Filtration and concentration gave trans-1-N-(4-fluorobenzamido)-6-nitroindan-2-ol(0.24g, 57%) as its hydrate, m.p. 208-210°C.

$^1$H nmr (DMSO- $d_6$):

δ 2.85 (dd,J=17 and 7.5Hz, 1H) 3.3 (dd, J=17 and 7 Hz, 1H); 3.5 (br, 2H); 4.5 (q, J=7 Hz, 1 H), 5.3 (t,J=7 Hz, 1H); 5.6 (br, 1H), 7.35 (t,J=7 Hz, 2H); 7.5 (d,J=8 Hz, 1H); 7.8 (br s, 1H); 8.00 (dd,J=7 and 2Hz, 2H); 8.1 (dd,J=8 Hz and 2 Hz, 1H); 8.9 (d,J=7 Hz, 1H)

| Anal: | | | | |
|---|---|---|---|---|
| $C_{16}H_{13}N_2O_4F.H_2O$ | requires<br>found | C,57.48;<br>C,57.58; | H,4.53;<br>H,4.19; | N,8.38.<br>N,8.25% |

Example 11

Trans-1-N-(4-chlorobutyrylamido)-6-nitroindan-2-ol

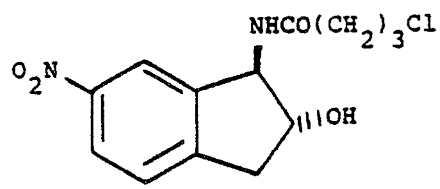

To a solution of trans-1-amino-6-nitroindan-2-ol (0.524g, 2.70mmol) in pyridine (20ml) at 0°C was added 4-chlorobutyrylchloride (0.26ml, 3.00mmol). The solution was stirred for 20 hours at room temperature, poured into 2N HCl (150ml) and extracted with ethyl acetate. The organics were separated, dried ($MgSO_4$) and concentrated in vacuo to a white solid. Chromatography on silica (ethyl acetate) yielded trans-1-N-(4-chlorobutyrylamido)-6-nitroindan-2-ol (0.334g, 42%)m.p. 140-141°C

$^1$H nmr ($CDCl_3$):

δ 2.25 (m, 2H); 2.55 (m, 2H), 3.05 (dd, J=17 and 8 Hz, 1 H) ; 3.4 (dd, J=17 and 8 Hz, 1H); 3.7 (t,J=6 Hz, 2H); 4.5 (m, 2H); 5.25 (app.t, J=6 Hz, 1 H); 6.1 (br, 1H); 7.4 (d, J=8 Hz, 1 H); 8.1 (brs, 1H) 8.2 (dd, J=8 and 2 Hz, 1 H).

| Anal: | | | | |
|---|---|---|---|---|
| $C_{13}H_{15}N_2O_4Cl$ | requires :-<br>found | C,52.26;<br>C,51.93; | H,5.07;<br>H,5.10; | N,9.38<br>N,9.54% |

Example 12

Trans-6-nitro-1-(2-oxopyrrolidin-1-yl)indan-2-ol

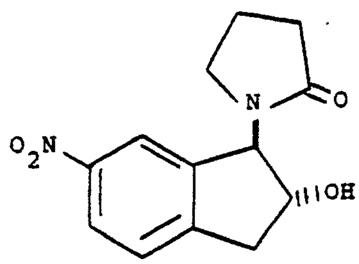

A mixture of trans-1-N-(4-chlorobutrylamido)-6-nitro-indan-2-ol(0.256g, 0.86mmol), potassium iodide (0.49g, 2.95mmol) and potassium carbonate (5.7g) in DMF was stirred at 50° C for 16 hours. The solution was concentrated and the residue partitioned between ethyl acetate and water. The organics were separated, dried ($MgSO_4$), concentrated and the crude product purified by preparative layer chromatography on silica (ethyl acetate/5% ethanol). Further purification was affected by refluxing in ethanol with decolourizing charcoal (ca.1.5g) for 4 hours which yielded trans-6-nitro-1-(2-oxopyrrolidin-1-yl)indan-2-ol m.p. 204-205° C (dec).

$^1$Hnmr (DMSO)$d_6$:

δ 2.0(m,2H); 2.4 (m, 2H); 2.8 (dd, J=16.5 and 8.5Hz, 1H); 3.05 (m,1H); 3.3 (m, 2H); 4.5 (app.q., J=7.4 Hz, 1H); 5.0 (br, 1H) ; 5.3 (d,J=7.4 Hz, 1H); 7.5 (d,J=8 Hz, 1H); 7.8 (brs, 1H); 8.1 (dd, J=8 and 1 Hz, 1 H).

| | | |
|---|---|---|
| Accurate Mass $C_{13}H_{14}N_2O_4$ | requires<br>found | 262.0954<br>262.0946 |

Example 13

Trans-3-(4-Fluorobenzoyl-N-methylamino)-1,1-dimethyl-5-nitroindan-2-ol

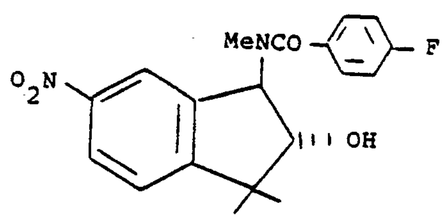

To a stirred solution of the trans-1,1-dimethyl-3-methylamino-5-nitroindan-2-ol of procedure 13 (0.32g,

1.35 mmol) and triethylamine (1.25ml, 9 mmol) in dichloromethane (50 ml) was added p-fluorobenzoyl chloride (0.215g, 1.36 mmol) dropwise. The solution was stirred for 1.5 h before washing with dil HCl and dil NaOH. The resulting organic solution was dried over anh. $MgSO_4$, filtered, evaporated and the yellow solid obtained recrystallised from ethyl acetate-pentane to give trans-3-(4-fluorobenzoyl-N-methylamino)-1,1-dimethyl-5-nitroindan-2-ol as a beige coloured solid (0.10g, 21%) of mp 184-6° C.

$^1$H nmr $CDCl_3$

δ 1.20(br s,3H); 1.36(s,3H); 2.90(s,3H); 4.18(d,J=10Hz,1H); 5.27 and 6.06(2 brm,1H); 7.15(m,2H); 7.38-(d,J=9Hz,1H); 7.67(m,2H); 8.00(narrow m,1H); 8.2(m,J=9Hz visible,1H).

Example 14

Trans-3-(4-Fluorobenzoyl-N-methylamino)-1,1-dimethyl-5-cyanoindan-2-ol

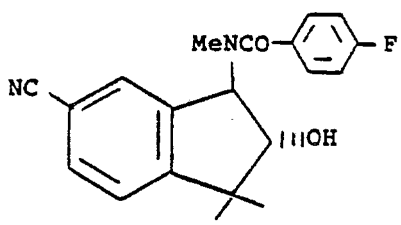

To a stirred solution of trans-1,1-dimethyl-3-methylamino-5-cyanoindan-2-ol from Procedure 14 (0.2g, 0.93 mmol) and triethylamine (0.3 ml, 2 mmol) in dichloromethane (20 ml) at 0° C, was added p-fluorobenzoyl chloride (0.15g, 0.95 mmol) dropwise. After the addition, the solution was stirred at room temperature for 1 h, then poured into dil. HCl. The organic layer was separated, dried ($MgSO_4$) and solvent removed in vacuo to give the title compound as a solid (0.28g, 89%) which recrystallised from 60-80° C petrol: ethyl acetate as needles (0.11g) having m.pt. 246-8° C.
Mass spectrum: $M^+$ 338.1429; $C_{20}H_{19}O_2N_2F$ requires $M^+$ 338.1429.

Examples 15 and 16

Trans-1,1-Dimethyl-3-(2-oxopiperidin-1-yl)-6-trifluoromethylindan-2-ol and 1,1-dimethyl-3-(2-oxopiperidin-1-yl)-6-trifluoromethylind-2-ene

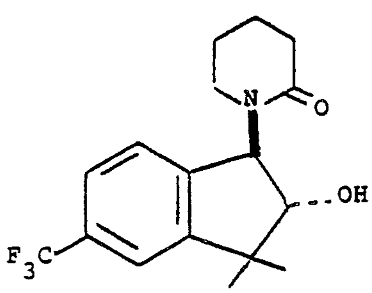

(Example 15)

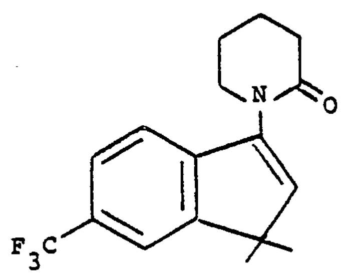

(Example 16)

Potassium t-butoxide (0.24g, 2.14mmol) was added to a solution of trans-2-bromo-1,1-dimethyl-6-trifluoromethylindan-3-ol (0.33g, 1.07mmol) in dimethylsulphoxide (5ml). After stirring for 15 minutes, 2-piperidone (0.53g, 5.35mmol) and potassium t-butoxide (0.6g, 5.35mmol) were added and the mixture stirred for a further 5.5 hours under nitrogen. The suspension was poured into hydrochloric acid solution (2M) and extracted with ethyl acetate. The organic phase was washed with brine, dried ($MgSO_4$) and concentrated to give a brown oil (300mg). Purification by chromatography on silica (chloroform then ethyl acetate) provided first 1,1-dimethyl-3-(2-oxopiperidin-1-yl)-6-trifluoromethylind-2-ene (8mg, 2%) followed by trans-1,1-dimethyl-3-(2-oxopiperidin-1-yl)-6-trifluoromethylindan-2-ol (65mg, 18%), m.p. 176-177° C.

Indanol (Example 15)

$^1$Hnmr ($CDCl_3$):

δ: 1.18 (s, 3H), 1.43 (s,3H), 1.86 (m, 4H), 2.59 (t, J=6.5Hz, 2H), 3.07 (d, J=6Hz, 1H), 3.12 (t, J=5.5Hz, 2H), 4.08 (dd, J=9.5, 6Hz, 1H), 6.17 (d, J=9.5Hz, 1H), 7.12 (d, 8Hz, 1H), 7.44 (br s, 1H), 7.48 (br d, J=8Hz, 1H).

| | | |
|---|---|---|
| Accurate Mass $C_{17}H_{20}N_2F_3$ | requires<br>found | 309.1341 ($M^+$-$H_2O$)<br>309.1348 |

Indene (Example 16)

$^1$H nmr ($CDCl_3$):

δ: 1.39 (s, 6H), 1.96 (m, 4H), 2.60 (m, 2H), 3.63 (m, 2H), 6.28 (s, 1H), 7.18 (d, J=8Hz, 1H), 7.48 (br d, J=8Hz, 1H), 7.53 (br s, 1H).

| | | |
|---|---|---|
| Accurate Mass $C_{17}H_{18}NOF_3$ | requires<br>found | 309.1341<br>309.1345 |

Example 17

Trans-1,1-Dimethyl-5-ethyl-3-(2-oxopiperidin-1-yl)indan-2-ol

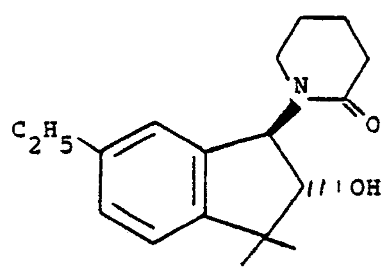

To a solution of trans-2-bromo-1,1-dimethyl-5-ethylindan-3-ol (500mg, 1.86mmol) in N-methylpyrrolidinone (8ml) was added 2-piperidone (921mg, 9.3 mmol) and potassium-tert- butoxide (1.04g, 9.3mmol). The mixture was stirred at room temperature under nitrogen for 16 hours, poured into 2N aqueous hydrochloric acid (50ml) and extracted with ethyl acetate (2x30ml). The organic phase was washed with water (20ml), dried ($MgSO_4$) and concentrated to a brown oil. Chromatography on silica (chloroform) afforded trans-1,1-dimethyl-5-ethyl-3-(2-oxopiperidin-1-yl)indan-2-ol as a white solid, m.p. 134-136° C.

$^1$H nmr ($CDCl_3$):

δ: 1.14 (s, 3H); 1.21 (t, J=7.7Hz, 3H); 1.22 (br, 1H)); 1.38 (s, 3H); 1.79-1.88 (m, 4H); 2.56 (m, 2H), 2.62 (q, J=7.7Hz, 2H); 3.15 (m, 2H), 4.01 (d, J=9Hz, 1H); 6.12 (d, J=9Hz, 1H); 6.81 (s, 1H); 7.11 (m, 2H).

| | | |
|---|---|---|
| Accurate Mass $C_{18}H_{25}NO_2$ | requires<br>found | 269.1780 ($M^+$-$H_2O$)<br>269. 1788 |

Examples 18 and 19

Trans-1,1-Dimethyl-6-pentafluoroethyl-3-(2-oxopiperidin-1-yl)indan-2-ol and 1,1-dimethyl-6-pentafluoroethyl-3-(2-oxopiperidin-1-yl)ind-2-ene

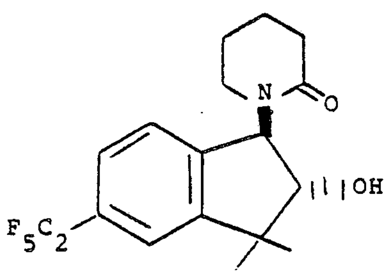

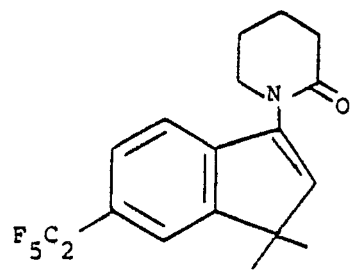

(Example 18) (Example 19)

A solution of trans-2-bromo-1,1-dimethyl-6-pentafluoroethylindan-3-ol (0.143g, 0.40mmol) and potassium t-butoxide (0.051g, 0.45mmol) in dimethylsulfphoxide (DMSO) (3ml) was stirred for 10 minutes and 2-piperidone (0.5ml, 5.01mmol) added followed by the portionwise addition of potassium t-butoxide (0.204g, 1.82mmol). The solution was stirred for 4.5 hours, poured into 2M HCl solution (30ml) and extracted with ethyl acetate (50ml). The organic portion was separated, dried and concentrated. Preparative layer chromatography (ethyl acetate/hexane 3:2) gave 1,1-dimethyl-6-pentafluoroethyl-3-(2-oxopiperidin-1-yl)ind-2-ene followed by trans-1,1-dimethyl-6-pentafluoroethyl-3- (2-oxopiperidin-1-yl)indan-2-ol as oils.

Indanol: (Example 18)

$^1$Hnmr ($CDCl_3$):

δ 1.25 (s, 3H); 1.45 (s, 3H); 1.85 (m, 5H); 2.6 (m, 2H); 3.2 (m, 2H); 4.1 (d, J = 13Hz, 1H); 6.2 (d, J = 13Hz, 1H); 7.1 (d, J = 8Hz, 1H); 7.45 (brs, 1H), 7.45 (d, J = 8Hz, 1H).

| | | | |
|---|---|---|---|
| Mass spectrum: | $C_{18}H_{20}NO_2F_5$ | found<br>requires | 359.1304 ($M^+$-$H_2O$)<br>359.1309 |

Indene: (Example 19)

$^1$Hnmr ($CDCl_3$):

δ:1.40 (s, 6H); 1.95 (m, 4H); 2.6 (m, 2H); 3.65 (m, 2H); 6.3 (s, 1H); 7.4 (m, 3H).

| | | | |
|---|---|---|---|
| Mass spectrum: | $C_{18}H_{18}NOF_5$ | found<br>requires | 359.1308<br>359.1309 |

Example 20

Trans-1,1-Dimethyl-3-(2-pyridon-1-yl)-6-trifluoromethylindan-2-ol

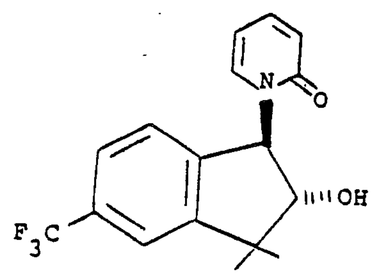

Potassium t-butoxide (558 mg, 4.97mmol) and 2-pyridone (316mg, 3.32mmol) were added to a solution of trans-2-bromo-1,1-dimethyl-6-trifluoromethylindan-3-ol (515mg, 1.66mmol) in dimethylsulfphoxide (8ml). The reaction mixture was stirred for 16.75 hours under nitrogen then poured into hydrochloric acid solution (2M) and extracted with ethyl acetate. The organic phase was washed with water, dried ($MgSO_4$) and concentrated to give an orange oil (450mg). Purification by preparàtive liquid chromatography on silica (ethyl acetate provided trans-1,1-dimethyl-3-(2-pyridon-1-yl)-6-trifluoromethylindan-2-ol.

$^1$Hnmr ($CDCl_3$):

δ 1.25 (s, 3H), 1.47 (s, 3H), 4.11 (d, 8.5Hz, 1H), 4.36 (m, 1H), 6.26 (dt, J = 7 and 1.5Hz, 1H), 6.40 (d, J = 8.5Hz, 1H), 6.67 (d, J = 9HZ, 1H), 7.08 (d, J = 8Hz, 1H), 7.17 (dd, J = 7 and 2Hz, 1H), 7.35-7.60 (m, 3H).

| | | |
|---|---|---|
| Accurate Mass $C_{17}H_{16}F_3O_2N$ | found<br>requires | 305.1024 [$M^+$ - $H_2O$]<br>305.1027 |

Example 21

Trans-1,1-Dimethyl-6-ethyl-3-(2-oxopiperidin-1-yl)indan-2-ol

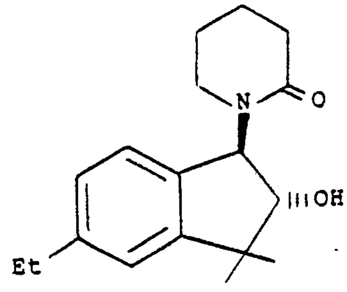

Under nitrogen, trans-2-bromo-1,1-dimethyl-6-ethylindan-3-ol (1.0g) was added to a solution of 2-piperidone anion (from 2-piperidone (1.86g) and potassium t-butoxide (2.13g)) in N-methylpyrrolidinone (14ml). The mixture was stirred overnight and aqueous workup yielded an oil (643mg). This was triturated with ethyl acetate/hexane (1:10) to give the title compound mp 160 °C (ethyl acetate).

$^1$H nmr ($CDCl_3$):

δ: 1.16 (s, 3H); 1.22 (t, J=7.5Hz, 3H); 1.38 (s, 3H); 1.80 (m, 4H); 2.57 (m, 2H); 2.63 (q, J=7.5Hz, 2H); 3.13 (m, 2H); 3.5 (bs, 1H); 4.00 (d, J=9Hz, 1H); 6.13 (d, J=9Hz, 1H); 6.8-7.2 (m, 3H).

IR (nujol)

$\nu_{max}$ $cm^{-1}$: 3410, 1630, 1110, 1035, 980, 960, 920, 890, 835.

| | | |
|---|---|---|
| Accurate Mass $C_{18}H_{25}NO_2$ | found | 269.1788 ($M^+$-$H_2O$) |
| | requires | 269.1780 |

Example 22

Trans-6-Cyano-1,1-dimethyl-3-(2-oxopiperidin-1-yl)indan-2-ol

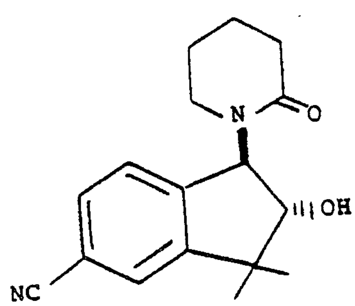

Under nitrogen, trans-2-bromo-6-cyano-1,1-dimethylindan-3-ol (347mg) was added to a solution of the anion of 2-piperidone (prepared from 2-piperidone (650mg), potassium t-butoxide (730mg)) in N-methylpyrrolidinone, (6 ml). The mixture was stirred overnight and aqueous work-up then yielded a gum. This was purified by chromatography on silica (ether/methanol (95:5)) to yield the title compound, mp 191 °C (ethyl acetate/hexane).

$^1$Hnmr ($CDCl_3$):

δ: 1.37 (s, 3H); 1.43 (s, 3H); 1.87 (m, 4H); 2.57 (m, 2H); 3.10 (m, 2H); 4.10 (d, J=9Hz, 1H), 6.13 (d, J=9Hz, 1H); 7.10 (d, J=7.5Hz, 1H); 7.43 - 7.60 (m, 2H)

IR (nujol)

$\nu_{max}$ $cm^{-1}$ 3395, 2220, 1620, 1110, 970, 920, 890, 865, 845.

Example 23

Trans 1,1-Dimethyl-5-ethyl-3-(2-pyridon-1-yl)indan-2-ol

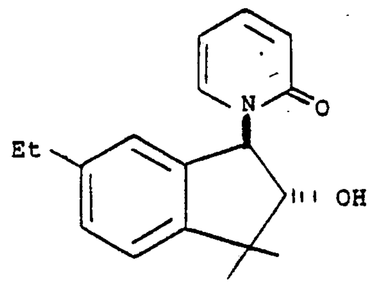

A mixture of trans-2-bromo-1,1-dimethyl-5-ethylindan-3-ol (1g, 3.7mmol), 2-pyridone (1.8g, 19mmol) and potassium tert-butoxide (2.08g, 18.5mmol) in N-methylpyrrolidinone (15ml) was stirred at room temperature under nitrogen for 16 hours. The mixture was poured into 2M aqueous hydrochloric acid (70ml) and extracted with ethyl acetate (2 x 30ml). The combined organic phase was washed with water (20ml, dried ($MgSO_4$) and concentrated to afford a light brown oil (1g). Cromatography on silica (ethyl acetate) gave trans-1,1-dimethyl-5-ethyl-3-(2-pyridon-1-yl)indan-2-ol as an oil.

$^1H$ nmr ($CDCl_3$):

δ: 1.19 (t, J=7.7Hz, 3H); 1.21 (s, 3H); 1.36 (s, 3H); 2.58 (q, J=7.7Hz, 2H); 3.4 (br, 1H); 4.02 (d, J=8.5Hz, 1H), 6.24 (dt, J=5 and 1.4Hz, 1H); 6.42 (d, J=8.2Hz, 1H); 6.68 (d, J=8.5Hz, 1H); 6.78 (s, 1H); 7.19 (m, 2H); 7.28 (m, 1H); 7.38 (m, 1H).

| | | |
|---|---|---|
| Accurate Mass $C_{18}H_{21}NO_2$ | requires<br>found | 265.1467 ($M^+$-$H_2O$)<br>265.1463 |

Example 24

5-Cyano-1,1-dimethyl-3-[(2-oxopyrrolidin-1-yl)methyl]ind-2-ene

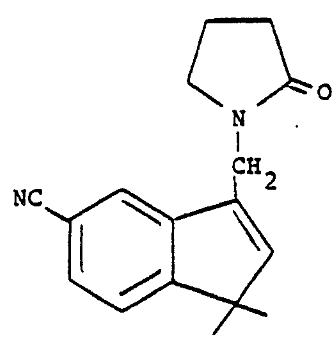

3-Bromomethyl-5-cyano-1,1-dimethylind-2-ene (0.316g, 1.21mmol) was added to a solution of pyrrolidinone (0.112g, 1.32mmol) in tetrahydrofuran (8ml) containing potassium t-butoxide (0.149g, 1.33mmol) and the mixture stirred for 72 hours. Water (10ml) was added to the reaction mixture which was then extracted with ethyl acetate. The organics were separated, dried ($MgSO_4$) and concentrated in vacuo. Preparative layer chromatography on silica (ethyl acetate) yielded 5-cyano-1,1-dimethyl-3-[(2-oxopyrrolidin-1-yl)methyl]ind-2-ene(0.16g, 50%), m.p. 102-103 °C.

$^1H$nmr ($CDCl_3$):

δ 1.3 (s,6H); 2.05 (m,2H); 2.45 (t, J=8.5Hz, 2H); 3.25 (t,J=6.5Hz, 2H); 4.4 (s,2H); 6.3 (s, 1H); 7.4 (d,J=8Hz, 1H); 7.5 (dd, J=8 ad 0.5 Hz, 1H); 7.6 (br s, 1H)

| Analysis: | | | | |
|---|---|---|---|---|
| $C_{17}H_{18}N_2O$ | requires :<br>found: | C,76.65;<br>C,76.87; | H,6.83;<br>H,6.84; | N,10.52%<br>N,10.51% |

Example 25

5-Cyano-1,2-dimethyl-3-[(2-pyridon-1-yl)methyl]ind-2-ene

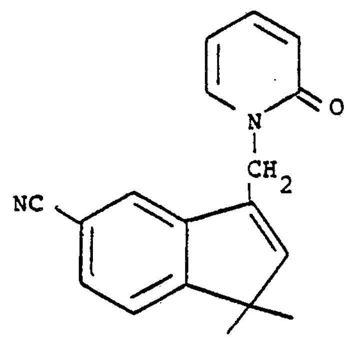

To the preformed anion of 2-pyridone [from 2-pyridone (0.067g, 0.71mmol) and potassium t-butoxide (0.077g, 0.69mmol)] in tetrahydrofuran (5ml) was added 3-bromomethyl-5-cyano-1,1-dimethylind-2-ene (0.160g, 0.61mmol) and the solution stirred for 56 hours after which time it was poured into water (15ml) and ethyl acetate (20ml). The organics were separated, dried and concentrated to a colourless oil. Preparative layer chromatography on silica (3:1 ethyl acetate/hexane) yielded 5-cyano-1,1-dimethyl-3-[(2-pyridon-1-yl)methyl]ind-2-ene (0.10g, 59%), m.p. 152 - 153° C.

$^1$Hnmr ($CDCl_3$):

δ 1.3 (s, 6H); 5.05 (d, J=1 Hz, 1 H); 6.2 (dt,J=7.5 and 1.5Hz, 1H); 6.30 (s, 1 H); 6.65 (d, J=6 Hz, 1 H); 7.3 (m, 3H); 7.55 (dd, J=7.5 and 1.5 Hz, 1H); 7.6 (d, J=0.6 Hz, 1H).

| Analysis: | | | | |
|---|---|---|---|---|
| $C_{18}H_{16}N_2O$ | requires :<br>found: | C, 78.22;<br>C, 78.61; | H, 5.85;<br>H, 5.87; | N, 10.14%<br>N, 9.90% |

Examples 26 and 27

Trans-1,1-Dimethyl-5-nitro-3-(5-(R)-methyl-2-oxopyrrolidin-1-yl)indan-2-ol and 1,1-dimethyl-5-nitro-3-(5-(R)-methyl-2-oxopyrrolidin-1-yl)ind-2-ene

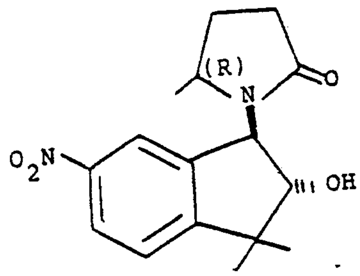

Example 26

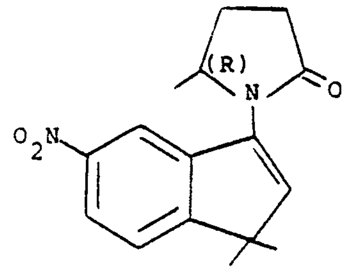

Example 27

Potassium t-butoxide (0.978g, 8.73mmol) was added to 5-(R)-methylpyrrolidinone (2.31g, 23.3mmol) and the solution stirred for 15 minutes whereupon a solution of 1,1-dimethyl-2,3-epoxy-5-nitroindane (1.47g, 7.17mmol) in DMSO (2ml) was added and the solution stirred for 1.5 hours. The reaction was quenched with 2M hydrochloric acid (30ml) and extracted with ethyl acetate (60ml). The organic portion was washed with water (3 x 50ml) dried and concentrated in vacuo. Chromatography on silica (gradient elution 2:1 ethyl acetate/hexane to ethyl acetate) yielded first starting epoxide (1.21g), followed by 1,1-dimethyl-5-nitro-3-(5-(R)-methyl-2-oxopyrrolidin-1-yl)ind-2-ene, m.p. 138-139°C, $[^{\alpha}D]^{25}$ EtOH -180.8° (0.063g) and finally trans-1,1-dimethyl-5-nitro-3-(5-(R)-methyl-2-oxopyrrolidin-1-yl)-indan-2-ol (0.12g) as a pair of diastereoisomers.

Indanols (Example 26)

$^1$H nmr ($CDCl_3$):

δ: 1.0 and 1.25 (d, J=6Hz, total 3H); 1.1 and 1.15 (s, total 3H); 1.4 and 1.45 (s, total 3H); 1.7 (m, 1H); 2.5 (m, 3H); 3.5 and 3.55 (d, J=5Hz, total 1H), 4.0 (m, 1H); 4.45 (m, 1H); 5.2 and 5.4 (d, J=8Hz, total 1H); 7.35 (m, 1H), ;7.85 and 7.9 (br s, total 1H), 8.15 (m, 1H).

Indene: (Example 27)

$^1$H nmr ($CDCl_3$):

δ: 1.23 (d, J=6Hz, 3H); 1.45 (s, 6H); 1.9 (m, 1H); 2.6 (m, 3H); 4.2 (m, 1H); 6.3 (s, 1H); 7.45 (d, J=8Hz, 1H); 8.05 (d, J=2Hz, 1H); ;8.15 (dd, J=8, 2Hz, 1H).

| | | | | |
|---|---|---|---|---|
| Analysis $C_{16}H_{18}N_2O_3$ | requires | C,67.10; | H,6.35; | N,9.78% |
| | found | C,67.10; | H,6.44; | N,9.66% |

Examples 28 and 29

Trans-1,1-Dimethyl-5-nitro-3-(5-(S)-methyl-2-oxopyrrolidin-1-yl)indan-2-ol and 1,1-dimethyl-5-nitro-3-(5-(S)-methyl-2-oxopyrrolidin-1-yl)ind-2-ene.

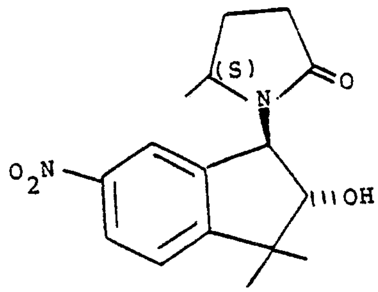

Example 28

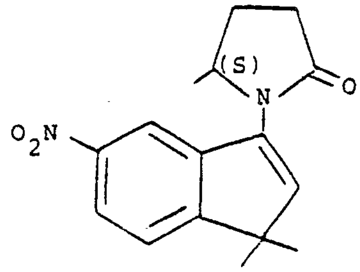

Example 29

Potassium t-butoxide (0.300g, 2.68mmol) was added to a solution of 5-(S)-methylpyrrolidinone (0.368g, 3.7 mmol) in DMSO (5ml) and the solution stirred for 10 minutes whereupon 1,1-dimethyl-2,3-epoxy-5-nitroindane (0.503g, 2.45mmol) was added and the solution stirred for 6 hours. The reaction mixture was poured into 2M hydrochloric acid (50ml) and extracted with ethyl acetate (60ml). The organic portion was separated, washed with water (3 x 50ml), dried and concentrated in vacuo. Chromatography on silica (2:1 ethyl acetate/hexane to ethyl acetate) yielded first 1,1-dimethyl-5-nitro-3-[5-(S)-methyl-2-oxopyrrolidin-1-yl]-ind-2-ene (0.077g) followed by trans-1,1-dimethyl-5-nitro-3-[5-(S)-methyl-2-oxopyrrolidin-1-yl)indan-2-ol (0.153g, 21%) as a pair of diastereoisomers.

Indanols (Example 28)

$^1$H nmr ($CDCl_3$):

δ: 1.0 and 1.25 (d, J=6Hz, total 3H); 1.1 and 1.15 (s, total 3H); 1.4 and 1.45 (s, total 3H); 1.75 (m, 1H); 2.25 (m, 3H) and 3.55 (d, J=8Hz, total 1H); 4.0 (m, 1H); 4.45 (m, 1H); 5.2 and 5.4 (d, J=8Hz, total 1H); 7.35 (m, 1H); 7.85 and 7.9 (br s, total 1H); 8.15 (m, 1H).

Indene (Example 29)

$^1$H nmr ($CDCl_3$):

δ: 1.23 (d, J=6Hz, 3H); 1.45 (s, 6H); 1.9 (m, 1H); 2.6 (m, 3H); 4.2 (m, 1H); 6.3 (s, 1H); 7.45 (d, J=8Hz, 1H); 8.05 (d, J=2Hz 1H); 8.15 (dd, J=8, 2Hz, 1H).

| | | |
|---|---|---|
| Accurate Mass $C_{16}H_{18}N_2O_3$ | requires<br>found | 286.1317<br>286.1307 |

Example 30

Trans-1,1-Dimethyl-5-iodo-3-(2-oxopiperidin-1-yl)indan-2-ol

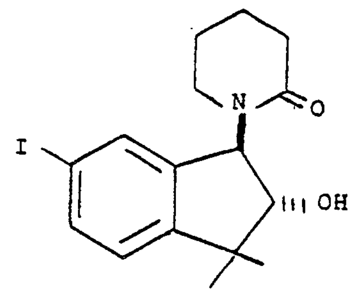

Trans-2-Bromo-1,1-dimethyl-5-iodoindan-3-ol (1g) in 1-methylpyrrolidin-2-one (12ml) was stirred with 2-piperidone (1.6g, 16mmol) and potassium tert butoxide (1.8g, 16mmol) at room temperature for 16 hours. The mixture was poured into 2M aqueous hydrochloric acid (70ml) and extracted with ethyl acetate (2 x 30ml). The combined organic phase was dried ($MgSO_4$), concentrated and separated by chromatography on silica (ethyl acetate/dichloromethane (7:3 v/v) to afford trans-1,1-dimethyl-5-iodo-3-(2-oxopiperidin-1-yl)-indan-2-ol as an oil.

$^1$H nmr ($CDCl_3$):

δ: 1.13 (s, 3H); 1.36 (s, 3H); 1.73-1.87 (m, 4H); 2.55 (m, 2H); 3.13 (m, 2H); 4.03 (d, J=9.3Hz, 1H); 4.20 (br, 1H); 6.12 (d, J=9.3Hz, 1H); 6.95 (d, J=8Hz, 1H); 7.21 (s, 1H); 7.57 (d, J=8Hz, 1H)

| | | |
|---|---|---|
| Accurate Mass: $C_{16}H_{17}INO$ | requires | 367.0433 [$M^+$-$H_2O$] |
| | found | 367.0416 |

Procedure 1

1,1-Dimethyl-5-nitroindan-3-ol

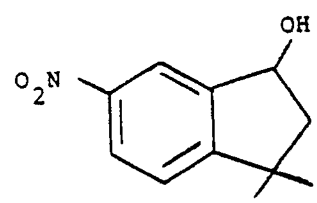

Potassium borohydride (1.03g, 19mmol) was added portionwise to a solution of 1,1 dimethyl-5-nitroindan-3-one (3.90g, 19mmol) in methanol (60ml) and the solution stirred for 1h. The solution was concentrated under vacuum and the residue partitioned between ethyl acetate and water. The organic phase was separated, washed with brine, dried ($MgSO_4$) and concentrated to an oil which crystallised on standing (3.76g, 95%). An analytical sample of 1,1-dimethyl-5-nitroindan-3-ol was obtained by recrystallisation from ether/hexane, m.p. 88-89 °C.

$^1$H n.m.r. ($CDCl_3$):

δ: 1.25 (s, 3H); 1.45 (s, 3H); 1.90 (d.d., J = 13, 6 Hz, 1H); 2.25 (d, J = 7 Hz, 1H); 2.34 (d.d. J = 13, 6.5 Hz, 1H); 5.35 (mm, 1H); 7.35 (d, J = 8Hz, 1H); 8.25 (m, 2H).

| Anal. | | | |
|---|---|---|---|
| Found<br>Requires | C, 63.87;<br>C, 63,74; | H, 6.37;<br>H, 6.34; | N, 6.83%<br>N, 6.76% for $C_{11}H_{13}NO_3$ |

Procedure 2

1,1-Dimethyl-5-nitroind-2-ene

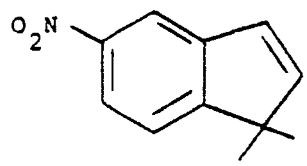

A solution of 1,1-dimethyl-5-nitroindan-3-ol (2.31g, 11.1mmol) in benzene (60ml) was heated at reflux in a Dean-Stark apparatus in the presence of p-toluene sulfphonic acid (150mg) for 16h. The solution was allowed to cool, washed with 2M sodium hydroxide solution, dried ($MgSO_4$) and concentrated to yield the required 1,1-dimethyl-5-nitroind-2-ene (1.90g, 90%) as a solid, m.p. 60 °C.

$^1H$ n.m.r. ($CDCl_3$):

δ 1.4 (s, 6H); 6.55 (d, J = 6 Hz, 1H); 6.7 (d, J=6Hz, 1H); 7.45 (d, J=9Hz, 1H); 8.15 m, 2H).

| Anal. | | | |
|---|---|---|---|
| Found<br>Requires | C, 69.44;<br>C, 69.81; | H, 5.89;<br>H, 5.87; | N, 7.49%<br>N, 7.40% for $C_{11}H_{11}NO_2$ |

Procedure 3

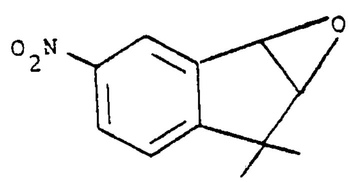

1,1-Dimethyl-2,3-epoxy-5-nitroindane

To a solution of 1,1-dimethyl-5-nitroind-2-ene (1.82g, 9.7mmol) in dichloromethane (40ml) was added m-chloroperbenzoic acid (2.06g, 1.1 equiv) portionwise. The solution was stirred for 48h, washed with saturated sodium sulphite solution then sodium bicarbonate solution, dried ($MgSO_4$) and concentrated *in vacuo*. Chromatographic purification on silica gel eluting with a 2:1 mixture of hexane/dichloromethane provided 1,1-dimethyl-2,3-epoxy-5-nitroindane (1.76g, 97%) as a cream solid, m.p. 50-53 °C.

$^1H$ n.m.r. ($CDCl_3$):

δ 1.3 (s, 3H); 1.5 (s, 3H); 3.8 (d, J=3Hz, 1H); 4.35 (d, J=3Hz, 1H); 7.4 (d, J=8Hz, 1H); 8.25 (d.d., J= 8, 3Hz, 1H); 8.35 (d, J=3Hz, 1H).

Procedure 4

6-Nitroindan-1-ol

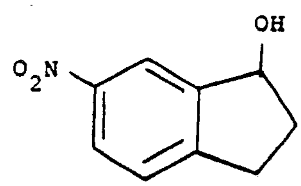

Potassium borohydride (2.80g, 52.8mmol) was added portionwise to a solution of 6-nitroindan-1-one (9.41g, 53.2mmol) in methanol (200ml) over 0.5 hours. The solution was stirred for 2 hours then concentrated. The residue was partitioned between ethyl acetate and water, and the organics isolated, dried ($MgSO_4$) then concentrated under reduced pressure. Chromatography on silica (1:1 ethyl acetate/hexane) yielded 6-nitroindan-1-ol (1.67g, 81%) as a cream solid which was used without further purification.

$^1$Hnmr ($CDCl_3$):

δ 2.0 (m, 1H), 2.35 (s, 1H), 2.55 (m, 1H), 2.95 (m, 1H), 5.3 (t, J=6 Hz, 1H), 7.35 (d, J=8 Hz, 1H), 8.15 (dd, J=8 and 2 Hz, 1H), 8.25 (d, J=2Hz, 1H).

Procedure 5

6-Nitroind-1-ene

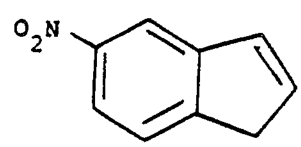

A solution of 6-nitroindan-1-ol (7.45g, 41.6mmol) in benzene (100ml) containing a catalytic amount of 4-toluenesulphonic acid (0.5g) was heated under reflux in a Dean-Stark apparatus for 16 hours. After cooling the solution was diluted with ether (100ml) and washed with saturated sodium bicarbonate solution. The organics were separated, dried ($MgSO_4$) and concentrated to a brown solid. Chromatography on silica (1:1 $CHCl_3$/hexane) provided 6-nitroind-1-ene (6.35g, 95%).

$^1$Hnmr ($CDCl_3$):

δ 3.55 (brt, 2H), 6.85 (m, 1H). 7.05 (m, 1H), 7.7 (d, J=8 Hz, 1H), 8.2 (dd, J=8, and 1.5Hz, 1H), 8.35 (d, J=1.5 Hz, 1H).

Procedure 6

1,2-Epoxy-6-nitroindane

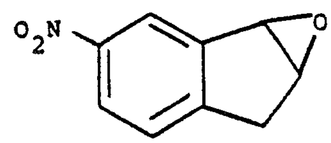

3-Chloroperbenzoic acid (2.89g, 13mmol) was added to a solution of 6-nitroind-1-ene (1.93g, 12 mmol) in dichloromethane (20ml) and the solution stirred for 2 days. The mixture was washed sequentially with saturated sodium bicarbonate solution, saturated sodium sulphite solution, saturated sodium bicarbonate solution, dried ($MgSO_4$) and concentrated in vacuo. Chromatography on silica (3:1 $CH_2Cl_2$/hexane) yielded 1,2-epoxy-6-nitroindane (1.82g, 86%), m.p. 95° C (ether/hexane).

$^1$Hnmr ($CDCl_3$):

δ 2,95 (dd, J=19 and 1.5Hz, 1H), 3.3 (d, J=19 Hz), 4.2 (brt,J=1.5 Hz, 1H), 4.35 (br d, J=3 Hz, 1H), 7.4 (d, J=8 Hz, 1H), 8.2 (dd, J=8 and 1.5Hz, 1H), 8.4 (d, J=1.5 Hz, 1H).

| Anal: | | | | |
|---|---|---|---|---|
| $C_9H_7NO_3$ | requires | C,61.01; | H,3.99; | N, 7.91 |
| | found | C,61.10; | H,3.94; | N, 7.84% |

Procedure 7

Trans-1-Azido-6-nitroindan-2-ol

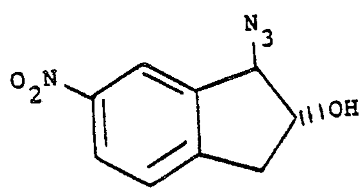

To a solution of 1,2-epoxy-6-nitroindane (5.27g, 29.8mmol) in dioxan (80ml) was added an aqueous solution (50ml) of sodium azide (2.87g, 44.2mmol). The solution was stirred for 20 hours, then extracted with ether. The organics were separated, dried ($MgSO_4$) and concentrated to a yellow oil (2.96g, 46%) which was used without further purification.

$^1$Hnmr ($CDCl_3$):

δ 2.65 (brs, 1H), 2.95 (dd, J=16.5 and 6 Hz, 1H), 3.4 (dd,J=16.5 and 7Hz, 1H), 4.55 (app q, J=6 Hz, 1H), 4.8 (d, J=6 Hz, 1H), 7.45 (d,J=9Hz, 1H), 8.25 (m, 2H).

Procedure 8

Trans-1-amino-6-nitroindan-2-ol

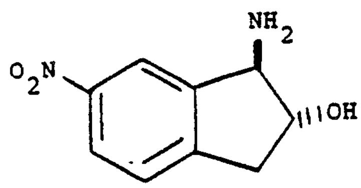

To a solution of trans-1-azido-6-nitroindan-2-ol (2.42g, 11mol) and sodium borohydride (2.36g, 62mmol) in THF at reflux was added methanol (10ml), dropwise, over 1 hour. The solution was heated for a further 3 hours cooled, diluted with brine and extracted with ethyl acetate. The organics were separated, dried ($MgSO_4$)and concentrated in vacuo. Chromatography on silica ($CHCl_3$/5%MeOH/0.5% Conc.$NH_3$) provided trans-1-amino-6-nitroindan-2-ol (0.93g, 43%), m.p. 136 - 137° C (EtOAc/hexane).

$^1$Hnmr ($CDCl_3$):

δ: 2.1 (br, 2H), 2.7 (dd, J=17 and 7 Hz, 1H), 3.2 (dd, J=17 and 6.5Hz, 1H), 4.0 (m,2H), 5.3 (br, 1H), 7.4 (d,J=8 Hz, 1H), 8.05 (dd, J=8 and 2 Hz, 1H), 8.1 (d,J=2Hz, 1H).

Procedure 9

1,1-Dimethyl-5-cyanoindan-3-one

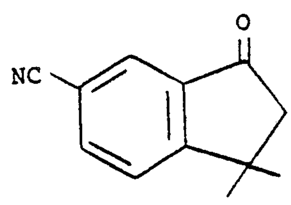

A solution of 1,1-dimethyl-5-aminoindan-3-one (1.0g, 5.7 mmol) in ethanol (5 ml) was added to conc. HCl (1.4 ml) in water (10 ml) at 0° C. To the cold mixture was added, dropwise, a solution of sodium nitrite (0.44g, 6.4 mmol) in water (3 ml), until an immediate positive reaction with starch-iodide paper was obtained. The reaction mixture was stirred for a further 15 min then added dropwise to a solution of potassium cyanide (1.48g, 22.8 mmol) and copper(1) cyanide (2.03g, 22.8 mmol) in water (20 ml) maintained at 90° C. On completion of addition, the reaction mixture was heated at 90° C for a further 25 min, then cooled, poured into water, and extracted with ethyl acetate. The organic extracts were dried ($MgSO_4$) and solvents removed in vacuo to give the title compound as a yellow solid (0.9g, 85%) which was used without further purification.

$^1$H n.m.r. ($CDCl_3$):

δ: 1.45(s,6H); 2.65(s,2H); 7.62(d,J=9Hz,1H); 7.78(dd,J=9,2Hz,1H); 7.98(d,J=2Hz,1H).

Procedure 10

1,1-Dimethyl-5-cyanoindan-3-ol

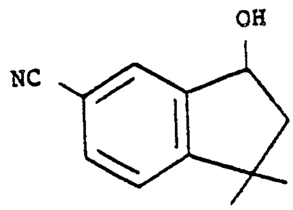

Sodium borohydride (0.185g, 4.9 mmol) was added to a stirred suspension of 1,1-dimethyl-5-cyanoindan-3-one (0.9g, 4.9 mmol) in ethanol (50 ml) at 0 °C, and the reaction mixture allowed to warm to room temperature overnight. The ethanol was then removed in vacuo and the residue partitioned between ethyl acetate and water. The organic extracts were dried ($MgSO_4$) and evaporated in vacuo to give the title compound as an oil (0.9g, 99%).

$^1$H n.m.r. ($CDCl_3$):

δ: 1.22(s,3H); 1.38(s,3H); 1.90(dd,J=12,6Hz,1H); 2.40(dd,J=12,6Hz,1H); 3.80(br s,1H); 5.25(m,1H); 7.25-(d,J=9Hz,1H); 7.55(m,2H).

Procedure 11

1,1-Dimethyl-5-cyanoind-2-ene

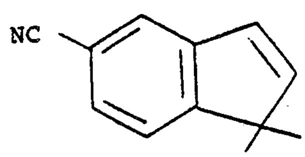

A solution of 1,1-dimethyl-5-cyanoindan-3-ol (0.9g, 4.8 mmol) in benzene (40 ml) was heated at reflux in a Dean-Stark apparatus in the presence of p-toluenesulphonic acid (100 mg) for 16 h. The solution was allowed to cool, washed with sodium hydrogen carbonate solution, brine, and dried ($MgSO_4$). Solvent was removed in vacuo and the residue chromatographed on silica gel eluting with 10% $CHCl_3$ in pentane to give the title compound (0.57g, 70%).

$^1$H n.m.r. ($CDCl_3$):

δ: 1.30(s,6H); 6.40(d,J=6Hz,1H); 6.55(d,J=6Hz,1H); 7.40(m,3H).

Procedure 12

1,1-Dimethyl-2,3-epoxy-5-cyanoidane

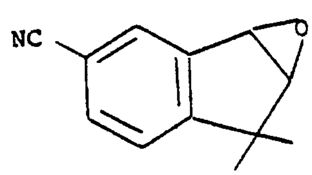

To a solution of 1,1-dimethyl-5-cyanoind-2-ene (0.57g, 3.4 mmol) in dichloromethane (30 ml) was added

m-chloroperbenzoic acid (0.8g, 1.1 equiv.). After stirring for 48 h, the solution was washed with saturated sodium sulphite solution then sodium bicarbonate solution, dried ($MgSO_4$) and evaporated in vacuo to give the required epoxide as a gum (0.62g, 99%).

$^1$H n.m.r. ($CDCl_3$):

δ: 1.20(s,3H); 1.40(s,3H); 3.68(d,J=3Hz,1H); 4.22(d,J=3Hz,1H); 7.18(d,J=8Hz,1H); 7.50(dd,J=8, 2Hz,1H); 7.60(d,J=2Hz,1H).

Procedure 13

Trans-1,1-Dimethyl-3-methylamino-5-nitroindan-2-ol

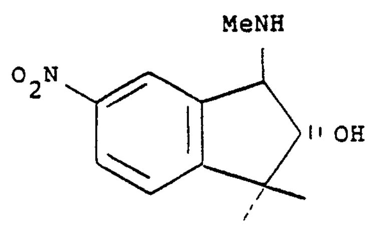

A solution of 1,1-dimethyl-2,3-epoxy-5-nitroindane (0.41g, 2.0 mmol) and methylamine (4 ml) in ethanol (100 ml) was stirred at room temperature for 24 h. The solution was evaporated and taken up in ethyl acetate and extracted with dil HCl. The aqueous phase was basified with dil NaOH solution and extracted with ethyl acetate. The organic solution was washed with water, dried with anh. $MgSO_4$, filtered and evaporated to give the methylaminoindane as a yellow solid (0.30g, 68%).
Mass spectrum (EI) $M^+$ at m/z 236.

Procedure 14

Trans-1,1-Dimethyl-3-methylamino-5-cyanoindan-2-ol

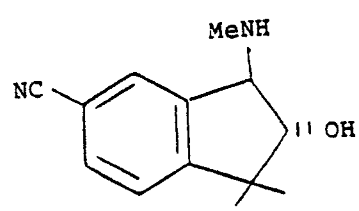

A solution of 1,1-dimethyl-2,3-epoxy-5-cyanoindane (0.62g, 3.35 mmol) and methylamine (1 ml) in ethanol (30 ml) was stirred at room temperature for 24 h. The solution was evaporated in vacuo. The residue was dissolved in ether and extracted with dil. HCl. The aqueous extracts were basified with NaOH and extracted with ethyl acetate. The organic phase was dried ($MgSO_4$) and evaporated in vacuo to give the required methylaminoindanol as a solid (0.2g), which was used directly in the next step.

Procedure 15

1,1-Dimethyl-6-trifluoromethylind-2-ene

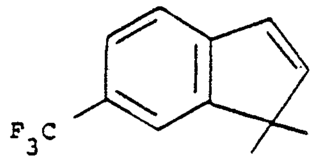

Cuprous iodide (4.58g, 24mmol) and sodium trifluoroacetate (3.26g, 24mmol) were added to a solution of 6-bromo-1,1-dimethylind-2-ene (1.34g, 6mmol) in 1-methyl-2-pyrrolidinone (60ml). The reaction mixture was stirred under nitrogen for 5.5 hours at 160° C, allowed to cool then water and diethylether (1:1) were added. After filtration using glass fibre paper and celite, the organic phase was washed with water, dried ($MgSO_4$) and concentrated to give a volatile brown liquid (1.6g) which was used without further purification.

$^1$H nmr ($CDCl_3$):

δ: 1.32 (s, 6H), 6.53 (d, J=6Hz, 1H), 6.68 (d, J=6Hz, 1H), 7.38 (d, J=9Hz, 1H), 7.55 (br d, J=9Hz, 1H), 7.6 (br s, 1H).

Procedure 16

Trans-2-Bromo-1,1-dimethyl-6-trifluoromethylindan-3-ol

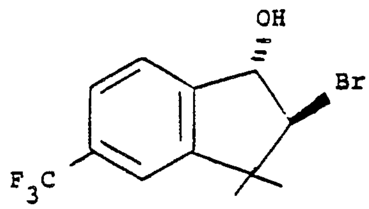

A solution of 1,1-dimethyl-6-trifluoromethylind-2-ene (6mmol) in dimethylsulphoxide (12ml), cooled in an ice-bath, was treated with N-bromosuccinimide (2.13g, 12mmol) and water (0.213ml, 13.2mmol). The solution was stirred at room temperature for 24 hours, poured into water and extracted with ethyl acetate. The organic phase was washed with water, dried ($MgSO_4$), concentrated and the crude product (1.87g) purified by chromatography on silica (chloroform/40% hexane). Further purification was affected by crystallisation from hexane and sublimation (100° C - 3mmHg) which yielded trans-2-bromo-1,1-dimethyl-6-trifluoromethylindan-3-ol (1.0g, 55%), m.p. 84-85° C.

$^1$H nmr ($CDCl_3$):

δ: 1.25 (s, 3H), 1.42 (s, 3H), 2.58 (m, 1H), 4.02 (d, J=9Hz, 1H), 5.29 (br d, J=9Hz, 1H), 7.43-7.65 (m, 3H).

| | | |
|---|---|---|
| Accurate Mass $C_{12}H_{12}F_3OBr$ | found<br>requires | 308.0018<br>308.0024 |

Procedure 17

5-Bromo-1,1-dimethylindan-3-ol

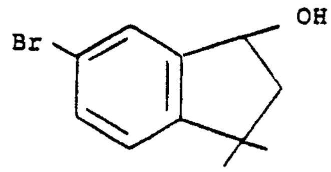

Sodium borohydride (1.84g, 48mmol) was added portionwise to a solution of 1,1-dimethyl-5-bromoindan-3-one (10.6g, 44 mol) in ethanol (300 ml) at 5° C and the solution stirred for 90 minutes at room temperature. The solution was poured into water (1200ml) and extracted with dichloromethane (3x150ml). The combined organic phase was dried ($MgSO_4$) and concentrated to provide 5-bromo-1,1-dimethylindan-3-ol as an oil which crystallised on standing (10.14g, 95%).

$^1$H nmr ($CDCl_3$):

δ: 1.20 (s, 3H),; 1.22 (s, 3H); 1.83 (dd, J = 14,6Hz, 1H); 2.33 (s, 1H); 2.36 (dd. J = 14,6Hz, 1H); 5.25 (m, 1H); 7.05 (d, J = 8Hz, 1H); 7.50 (dd. J = 8,2Hz, 1H); 7.60 (d, J = 2Hz, 1H).

Procedure 18

5-Bromo-1,1-dimethylind-2-ene

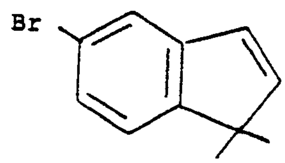

A solution of 5-bromo-1,1-dimethylindan-3-ol (10g, 41mmol) in toluene (350ml) was heated at reflux under nitrogen in a Dean-Stark apparatus in the presence of p-toluenesulphonic acid (300mg) for 2.5 hours. The solution was allowed to cool, filtered and concentrated to a brown oil. Chromatography on silica (hexane) afforded 5-bromo-1,1-dimethylind-2-ene (7.3g, 79%) as a colourless oil.

$^1$H nmr ($CDCl_3$):

δ: 1.25 (s, 6H); 6.35 (d, J = 5Hz, 1H); 6.55 (d, J = 5Hz, 1H); 7.15 (d, J = 8Hz, 1H); 7.32 (dd, J = 2,8Hz, 1H); 7.43 (d, J = 2Hz, 1H).

Procedure 19

1,1-Dimethyl-5-ethylind-2-ene

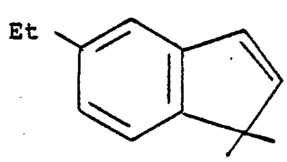

To 5-bromo-1,1-dimethylind-2-ene (2g, 9mmol) in dry diethyl ether (20ml) at room temperature under nitrogen was added bis(diphenylphosphino)ethane nickel (II) chloride (47mg, 0.09mmol). Ethyl magnesium

bromide (3.5ml of a 3M solution in diethyl ether, 10.5mmol) was added over 10 minutes and the mixture heated at reflux for 16 hours. After cooling, the solution was added to 2N aqueous hydrochloric acid (10ml) and the phases separated. The aqueous phase was extracted with diethyl ether (20ml), the combined organic phase dried ($MgSO_4$) and concentrated to afford 1,1-dimethyl-5-ethylind-2-ene (1.54g, 100%) as an oil.

$^1$H nmr ($CDCl_3$):

δ: 1,20 (m, 9H); 2.53 (q, J=8Hz, 2H); 6.27 (d, J=5Hz, 1H); 6.53 (d, J=5Hz, 1H); 6.93-7.27 (m, 3H).

Procedure 20

Trans-2-Bromo-1,1-dimethyl-5-ethylindan-3-ol

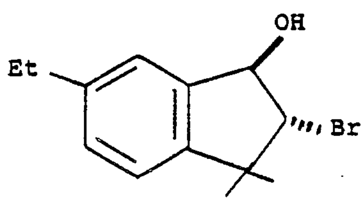

N-Bromosuccinimide (2.9g, 16.4mmol) was added to a solution of 1,1-dimethyl-5-ethylind-2-ene (1.42g, 8.2mmol) in dimethylsulphoxide (18ml) and water (0.4ml). The mixture was stirred at room temperature for 3 days, poured into water (200ml) and extracted with ethyl acetate (100ml). The organic phase was washed with water (50ml), dried ($MgSO_4$) and concentrated to afford trans-2-bromo-1,1-dimethyl-5-ethylindan-3-ol (2.19g, 100%) as an oil which crystallised on standing.

$^1$H nmr ($CDCl_3$):

δ: 1.03 (s, 3H); 1.06 (t, J=8Hz, 3H); 1.20 (s, 3H); 2.50 (q, J=8Hz, 2H); 3.76 (d, J=8Hz, 1H); 3.80 (br.s, 1H), 5.03 (d, J=8Hz, 1H), 7.00-7.20 (m, 3H).

Procedure 21

1,1-Dimethyl-6-trifluoroacetylind-2-ene

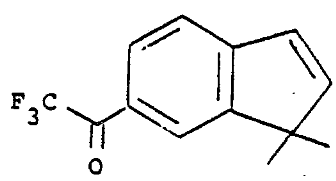

A mixture of 6-bromo-1,1-dimethylind-2-ene (0.375g, 1.68mmol), magnesium turnings (45mg, 1.88mmol) and iodine ( 1mg) in tetrahydrofuran (10ml) was heated at reflux for 1 hour until all the magnesium had reacted. The reaction mixture was then cooled to 0°C and trifluoroacetic anhydride (0.26ml, 1.83mmol) added and stirring continued for a further 1 hour. The reaction was quenched with saturated sodium bicarbonate solution (30ml) and extracted with ether. The organic portion was separated, dried and concentrated to an oil. Chromatography on silica (9:1 hexane/$CH_2Cl_2$) gave 1,1-dimethyl-6-trifluoroacetylind-2-ene (0.196g, 49%) as a yellow liquid.

$^1$H nmr ($CDCl_3$):

δ: 1.4 (s, 6H); 6.7 (s, 2H); 7.45 (d, J=8Hz, 1H); 8.05 (m, 2H).

Procedure 22

1,1-Dimethyl-6-pentafluoroethylind-2-ene

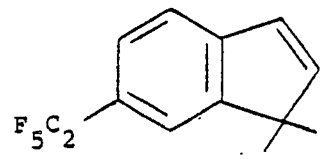

A solution of 1,1-dimethyl-6-trifluoroacetylind-2-ene (0.46g, 1.92mmol), and diethylaminosulphur trifluoride (0.38ml, 2.83mmol) in $CH_2Cl_2$ (10ml) was stirred for 16 hours then poured into water. The organic portion was separated, dried and concentrated. Chromatography on silica (hexane) gave 1,1-dimethyl-6-pentafluoroethylind-2-ene (0.296g, 59%) as a colourless liquid.

$^1$H nmr ($d_6$-DMSO):

δ: 1.4 (s, 6H); 6.7 (d, J=5Hz, 1H); 6.8 (d, J=5Hz, 1H); 7.55 (br s, 2H), 7.75(br s, 1H).

Procedure 23

Trans-2-Bromo-1,1-dimethyl-6-pentafluoroethylindan-3-ol

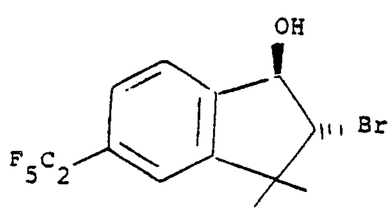

Method A

To a solution of 1,1-dimethyl-6-pentafluoroethylind-2-ene (0.285g, 1.09mmol) in DMSO (7ml) was added 5 drops of water followed by N-bromosuccinimide (0.425g, 2.40mmol) and the solution stirred for 3 hours. The reaction mixture was poured into ethyl acetate (60ml)/water (50ml) and the organic portion separated washed with water (3 x 50ml), dried and concentrated in vacuo. Chromatography of the residue on silica (3:2 $CHCl_3$/hexane) gave trans-2-bromo-1,1-dimethyl-6-pentafluoroethylindan- 3-ol (0.337g, 86%) as a white solid, m.p. 73-74°C (hexane).

Method B

A mixture of cuprous iodide (7.28g, 38.3mmol), sodium pentafluoropropionate (7.23g, 38.9mmol) and 1,1-dimethyl-6-bromoind-2-ene (2.01g, 9.0mmol) in N-methylpyrrolidinone (20ml) was heated at 170°C for 5.5 hours. The mixture was allowed to cool, diluted with ether (150ml) and filtered through a pad of celite. The supernatant was washed with water (3 x 100ml), dried and concentrated. The crude 1,1-dimethyl-6-pentafluoroind-2-ene was dissolved in DMSO (40ml) and water (1.5ml) and N-bromosuccinimide (2.96g, 16.6mmol) added. The solution was stirred overnight, diluted with water (150ml) and extracted with ether (100ml). The organics were separated, washed with water (3 x 100ml), dried and concentrated. Chromatography of the residue on silica (3:2 $CHCl_3$/hexane) gave trans-2-bromo-1,1-dimethyl-6-pentafluoroindan-3-ol (1.28g, 39%).

$^1$H nmr ($CDCl_3$):

δ: 1.25 (s, 3H); 1.4 (s, 3H,); 2.6 (br, 1H); 3.95 (d,J=9Hz, 1H); 5.3 (d, J=9Hz 1H); 7.45 (br s, 1H); 7.55 (br s, 2H).

| | | |
|---|---|---|
| Accurate Mass $C_{13}H_{12}BrF_5O$ | found | 357.9998 |
| | requires | 357.9992 |

Procedure 24

6-Bromo-1,1-dimethylindan-3-ol

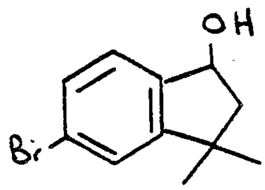

6-Bromo-1,1-dimethylindan-3-one (J. Smith and M. Massicotte, Org. Prep. and Proc., 10, 123, 1978) (8.5g, 36mmol) in ethanol (400ml) was cooled to 0°C. Sodium borohydride (1.85g, 49mmol) was added over 15 minutes with stirring. The mixture was stirred for 3 hours, the solvent evaporated and aqueous workup yielded the title compound as a white gum, 8.6g (100%).

$^1$H nmr ($CDCl_3$):

δ: 1.21 (s, 3H); 1.38 (s, 3H); 1.78 (s, 1H); 1.83 (dd, J=13 and 7Hz, 1H); 2.40 (dd, J=13 and 7Hz, 1H); 5.23 (t, J=7Hz, 1H); 7.37 (m, 3H).

IR (film)

$\nu_{max}$ cm$^{-1}$: 3210, 1595, 1580, 970, 870, 815, 790, 760.

Procedure 25

6-Bromo-1,1-dimethylind-2-ene

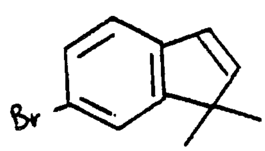

6-Bromo-1,1-dimethylindan-3-ol (8.6g) was dissolved in toluene (400ml) and treated with 4-toluenesulphonic acid (20mg). The mixture was refluxed overnight under Dean and Stark conditions and the solution washed with sodium bicarbonate, water and brine. Removal of the toluene yielded the title compound (7.8g, 98%).

$^1$H nmr ($CDCl_3$):

δ: 1.29 (s, 6H); 6.40 (d, J=6Hz, 1H); 6.65 (d, J=6Hz, 1H); 7.16 to 7.60 (m, 3H).

IR (film) .

$^{\nu}$max cm$^{-1}$: 3040, 1560, 940, 870, 850, 815, 770, 725.

Procedure 26

1-1-Dimethyl-6-ethylind-2-ene

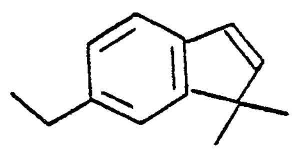

6-Bromo-1,1-dimethylind-2-ene (2.00g) was combined with magnesium (0.225g) in dry tetrahydrofuran (THF, 50ml) under nitrogen and stirred until the magnesium had reacted. Ethyl bromide (1.65g) was added followed by four drops of a solution of LiCl (850mg) and dry cupric chloride (1.35g) in THF (5ml). The mixture was stirred overnight and worked up using ammonium chloride solution to give 1,1-dimethyl-6-ethylind-2-ene (735mg) containing some 1,1-dimethylind-2-ene.

$^1$H nmr ($CDCl_3$):

δ: 1.25 (t, J=7.5Hz, 3H); 1.32 (s, 6H); 2.70 (q, J=7.5Hz, 2H); 6.33 (d, J=5Hz, 1H); 6.65 (d, J=5Hz, 1H); 7.0-7.33 (m, 3H).

IR (film)

$^{\nu}$max cm$^{-1}$: 3025, 1610, 1390, 995, 885, 870, 830, 800, 750, 735.

Procedure 27

Trans-2-Bromo-1,1-dimethyl-6-ethylindan-3-ol

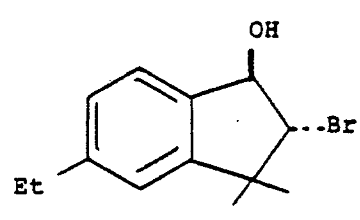

The crude 1,1-dimethyl-6-ethylind-2-ene from Procedure 26 (700mg) was combined with DMSO (9ml),

water (180mg) and N-bromosuccinimide (1.51g) and stirred overnight, Aqueous workup followed by chromatography on silica (ethyl acetate/hexane (1:10)) yielded trans-2-bromo-1,1-dimethyl-6-ethylindan-3-ol as a gum, 1.023g.

$^1$H nmr (CDCl$_3$):

δ: 1.21 (s, 3H); 1.25 (t, J=7.5Hz, 3H); 1.37(s, 3H); 2.60 (s, 1H); 2.67 (q, J=7.5Hz, 2H); 3.95 (d, J=8Hz, 1H); 5.22 (d, J=8Hz, 1H); 7.0-7.4 (m, 3H).

IR (film)

$\nu_{max}$ cm$^{-1}$: 3320, 1600, 1460, 885, 830, 785, 730.

Procedure 28

6-Cyano-1,1-dimethylindan-3-one

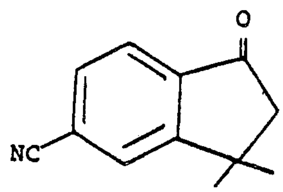

6-Bromo-1,1-dimethylindan-3-one (500mg) was combined with cuprous cyanide (256mg) and dimethylformamide (10ml). The solution was degassed under nitrogen and refluxed overnight. Aqueous workup yielded the title compound, 342mg, m.p. 126° C (hexane).

$^1$Hnmr (CDCl$_3$):

δ: 1.50 (s, 6H); 2.67 (s, 2H); 7.7-8.0 (m, 3H). $\nu_{max}$ cm$^{-1}$: 2220, 1725, 1600, 920, 840, 745, 730.

Procedure 29

6-Cyano-1,1-dimethylindan-3-ol

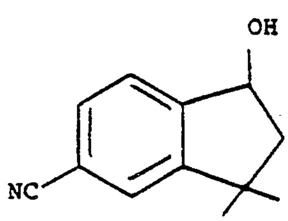

6-Cyano-1,1-dimethylindan-3-one (335mg) was stirred with sodium borohydride (95mg) in ethanol (20ml) for three hours. The solvent was evaporated and the residue taken up in ether (50ml) and washed with water (3 x 25ml). Evaporation of the ether yielded the title compound as an oil (338mg, 100%).

$^1$Hnmr (CDCl$_3$):

δ: 1.17 (s, 3H); 1.43 (s, 3H); 1.87 (dd, J=12, 7.5Hz, 1H); 2.05 (s, 1H); 2.47 (dd, J=12, 7.5Hz, 1H); 5.32 (t, J=7.5Hz, 1H); 7.53 (m, 3H).

IR (film)

$\nu_{max}$ $cm^{-1}$: 3310, 2220, 1590, 1580, 970, 870, 815, 790, 760, 730.

Procedure 30

6-Cyano-1,1-dimethylind-2-ene

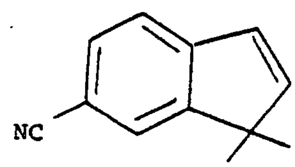

6-Cyano-1,1-dimethylindan-3-ol (338mg) was combined with toluene (20ml) and 4-toluenesulphonic acid (5mg). The mixture was heated under reflux overnight under Dean and Stark conditions. Aqueous work-up yielded the title compound as a gum, (268mg, 85%).

$^1$Hnmr ($CDCl_3$):

δ: 1.33 (s, 6H); 6.65 (m, 2H); 7.2-7.67 (m, 3H).

IR (film)

$\nu_{max}$ $cm^{-1}$: 2220, 1610, 950, 890, 835, 790, 740.

Procedure 31

Trans-2-Bromo-6-cyano-1,1-dimethylindan-3-ol

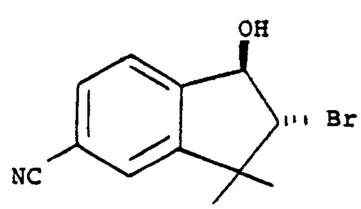

6-Cyano-1,1-dimethylind-2-ene (260mg) was combined with DMSO (3ml), water (72mg) and N-bromosuccinimide (534mg) and stirred overnight. Aqueous work-up yielded the title compound as a gum (347mg, 85%).

$^1$Hnmr ($CDCl_3$):

δ: 1.15 (s, 3H); 1.33 (s, 3H); 3.62 (bs, 1H); 3.93 (d, J=9Hz, 1H), 5.20 (d, J=9Hz, 1H); 7.4-7.73 (m, 3H).

IR (film)

$\nu_{max}$ $cm^{-1}$: 3380, 2220, 1630, 900, 885, 870, 825, 750, 725.

Procedure 32

5-Amino-1,1-dimethylindan-3-one

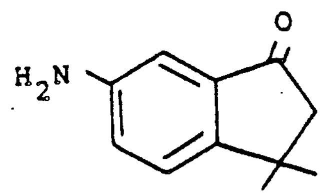

A solution of 1,1-dimethyl-5-nitroindan-3-one (0.94g, 4.59mmol) and 10% palladium on carbon (0.32g) in methanol (30ml) was hydrogenated until the required uptake had been achieved. The mixture was filtered, concentrated and the crude product purified by chromatography on silica (1:1 ethyl acetate/hexane) to yield 5-amino-1,1-dimethylindan-3-one (0.64g, 80%), m.p. 75 -76° C (ether/petrol).

$^1$Hnmr ($CDCl_3$):

δ: 1.4 (s,6H); 2.55 (s, 2H); 3.8 (brs, 2H); 6.95 (m,2H); 7.3 (dd, J=8 and 0.5Hz, 1H).

| Analysis: | | | | |
|---|---|---|---|---|
| $C_{11}H_{13}NO$ | requires :<br>found : | C, 75.38;<br>C, 74.64; | H,7.49 ;<br>H,7.42 ; | N, 7.99%<br>N, 8.06% |

Procedure 33

5-Amino-1,1-dimethyl-3-methyleneindane

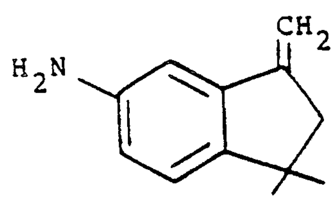

Potassium t-butoxide (6.04g, 53.9mmol) was added to a solution of methyltriphenylphosphonium bromide (19.03g, 53.3mmol) in tetrahydrofuran (100ml) at -78° C. After 10 min. 5-amino-1,1-dimethylindan-3-one (4.06g, 23.2mmol) in tetrahydrofuran (20ml) was added and the mixture allowed to attain ambient temperature and stirred for 72 hours, after which time brine was added and the solution extracted with ether. The organics were separated, dried and concentrated. Chromatography on silica ($CH_2Cl_2$) provided 5-amino-1,1-dimethyl-3-methyleneindane (3.85g, 96%) as a yellow liquid.

$^1$Hnmr ($CDCl_3$):

δ: 1.4 (s, 6H); 2.75 (t, J=2 Hz, 2H); 3.7 (brs, 2H); 5.1 (t, J=2 Hz, 1H); 5.5 (t,J=2 Hz, 1H); 6.75 (dd, J=8 and

2Hz, 1H); 6.9 (d, J=2Hz, 1H); 7.15 (d, J=8 Hz, 1H).

Procedure 34

5-Cyano-1,1-dimethyl-3-methyleneindane

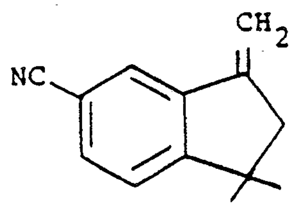

To 5-amino-1,1-dimethyl-3-methyleneindane, (3.24g, 18.7 mmol) in water (40ml) containing conc. hydrochloric acid (4.8ml) and ethanol (20ml) at 0° C was added, dropwise, sodium nitrite (1.4g, 20.2mmol) in water (10ml) until an immediate positive reaction was obtained with starch-iodide paper. The mixture was stirred at 0° C for a further 15 minutes then added dropwise to an aqueous solution 50ml) of potassium cyanide (5.6g, 83.6mmol) and cuprous cyanide (7.9g, 84.5mmol) at 90° C at such a rate that the temperature did not drop below 70° C. After the addition was completed heating was continued for a further 20 minutes and the reaction mixture cooled and extracted with ethyl acetate. The organics were dried, concentrated and chromatographed on silica (2:1 $CHCl_3$/hexane) to give 5-cyano-1,1-dimethyl-3-methyleneindane (1.62g, 47%) as an oil.

$^1$Hnmr ($CDCl_3$:

δ: 1.3 (s, 6H); 2.7 (t,J=2.5Hz, 1H); 5.15 (t,J=2 Hz, 1H); 5.5 (t,J=2.5Hz, 1H); 7.3 (d, J=8 Hz, 1H); 7.5 (dd, J=8 and 1.5 Hz., 1H); 7.7 (d, J=1.5Hz, 1H).

Procedure 35

5-Cyano-1-1,3-trimethylind-2-ene

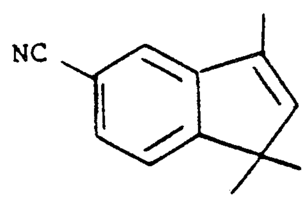

A solution of 5-cyano-1,1-dimethyl-3-methyleneindane (0.871g, 4.75mmol) and trifluoroacetic acid (0.5ml) in $CH_2Cl_2$ (10ml) was stirred for 7 hours, concentrated and the residue chromatographed on silica (1:1, $CH_2Cl_2$/hexane) to yield 5-cyano-1,1,3-trimethylind-2-ene (0.77g, 89%) as a liquid.

$^1$Hnmr ($CDCl_3$):

δ: 1.3 (s, 6H); 2.1 (d, J=1.5Hz, 3H); 6.15 (q, J=1.5 Hz,1H); 7.3 (dd,J=7.5 and 0.5Hz, 1H); 7.5 (m,2H).

Procedure 36

3-Bromomethyl-5-cyano-1,1-dimethylind-2-ene

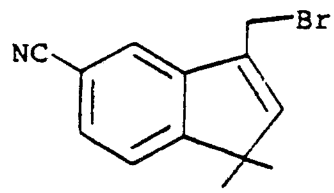

A solution of 5-cyano-1,1,3-trimethylind-2-ene (0.56g, 3.06mmol), N-bromosuccinimide (0.609g, 3.37mmol) and AIBN (10mg) in carbon tetrachloride (7ml) was irradiated with a 120W tungsten lamp for 18 hours. The solution was allowed to cool, filtered and concentrated. Chromatography of the residue on silica (4:1 hexane/ethyl acetate) provided 3-bromomethyl-5-cyano-1,1-dimethylind-2-ene (0.50g, 62%) as an oil.

$^1$Hnmr ($CDCl_3$):

δ: 1.3 (s, 6H); 4.35(d,J=0.8 Hz, 2H); 6.5(brs 1H); 7.4 (d, J=8 Hz, 1H0; 7.55 (dd, J=8 and 1.5 Hz, 1H); 7.7 (d,J=1.5Hz, 1H).

Procedure 37

Trans-2-Bromo-1,1-dimethyl-5-iodoindan-3-ol

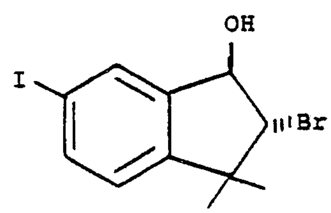

A mixture of 5-bromo-1,1-dimethylind-2-ene (400mg, 1.8mmol), cuprous iodide (2.13g, 11mmol) and potassium iodide (3.72g, 22.4mmol) in dimethylformamide (25ml) was heated at 160° C for 18 hours under nitrogen. The cooled solution was poured into 2M aqueous hydrochloric acid (50ml) and extracted with diethyl ether (2 x 30ml). The combined organic phase was ashed with aqueous sodium sulphite solution and water and dried ($MgSO_4$). Concentration afforded 1,1-dimethyl-5-iodoind-2-ene. The crude indene (2.3g) was dissolved in dimethylsulphoxide (18ml) and water (1ml) and stirred with N-bromosuccinimide (3.2g, 18mmol) at room temperature for 16 hours. The mixture was poured into water (300ml) and extracted with ethyl acetate (2 x 74ml). The combined organic phase was washed with water (50ml), dried ($MgSO_4$) and concentrated to afford the title compound (2.4g) which was used without purification.

Pharmacological Data

1. Bronchodilator Activity

(a) Bronchodilation in vitro; guinea pig tracheal spiral preparations.

Male guinea pigs (300-600g) were stunned by a blow to the head and bled from the carotid artery. The trachea was exposed, dissected free of connective tissue, and transferred to oxygenated krebs solution at 37° C. Next, spirals (2 per trachea) were prepared by cutting the whole trachea spirally along its longitudinal

axis and then dividing this spiral lengthwise. Each preparation was mounted, using silk thread, in a 10ml organ bath filled with krebs solution at 37°C and bubbled with 5% $CO_2$ with $O_2$. The resting tension of the preparations was set at 2g and changes in muscle tension were monitored isometrically by means of a UFI (2oz) force and displacement transducer (Ormed Ltd) connected to a Linseis pen recorder. All preparations were allowed to equilibrate for 60 minutes. During this equilibration period the preparations were washed by upward displacement at 15 minute intervals and, if necessary, the resting tension was readjusted to 2g using a mechanical micromanipulator system.

Once a steady resting tension had been obtained, the preparations were dosed cumulatively with the test compound ($10^{-8}$-$2x10^{-5}$M), and finally a maximum relaxation achieved by addition of $10^{-3}$M isoprenaline. The fall in tension evoked by the test compound was expressed as a percentage of the total relaxation evoked in the presence of $10^{-3}$M isoprenaline. Appropriate concentration-relaxation curves were then constructed and values for potency ($IC_{50}$) and efficacy (Intrinsic Activity, I.A.) were obtained.

The compositions of Krebs solution is: sodium chloride 118.07mM, sodium hydrogen carbonate 26.19mM, potassium chloride 4.68mM, potassium orthophosphate 1.18mM, magnesium sulphate septahydrate 1.8mM and calcium chloride 2.52mM;pH ca. 7.45.

| Results | | |
|---|---|---|
| Example No. | In vitro $IC_{50}$ (M) | I.A. |
| 1 | $3.1 \times 10^{-6}$ | 0.88 |
| 3 | $3.9 \times 10^{-7}$ | 0.89 |
| 4 | $2.78 \times 10^{-6}$ | 0.94 |
| 5 | $3.5 \times 10^{-7}$ | 0.91 |
| 6 | $8.7 \times 10^{-7}$ | 0.95 |
| 7 | $2.0 \times 10^{-6}$ | 0.81 |
| 8 | $5.3 \times 10^{-6}$ | 0.77 |
| 10 | $1.3 \times 10^{-5}$ | 0.59 |
| 16 | $8.55 \times 10^{-6}$ | 0.89 |
| 17 | $3.75 \times 10^{-6}$ | 0.76 |
| 23 | $2.60 \times 10^{-6}$ | 0.92 |
| 24 | $1.4 \times 10^{-5}$ | 0.59 |
| 26 | $2.6 \times 10^{-7}$ | 0.98 |
| 27 | $1.2 \times 10^{-6}$ | 0.93 |

2. Antihypertensive Activity

Blood Pressure Lowering Activity

Systolic blood pressure were recorded by a modification of the tail cuff method described by I.M. Claxton, M.G. Palfreyman, R.H. Poyser, R.L. Whiting, European Journal of Pharmacology, 37, 179 (1976). A W+W BP recorder, model 8005 was used to display pulses.Prior to all measurements rats were placed in a heated environment (33.5 ± 0.5°C) before transfer to a restraining cage. Each determination of blood pressure was the mean of at least 6 readings. Spontaneously hypertensive rats (ages 12-18 weeks) with systolic blood pressures >180 mmHg were considered hypertensive.

| Results | | | | |
|---|---|---|---|---|
| Compound of Example No | dose (mg/kg) p.o. | Time post dose (h) | % change in systolic B.P. | Initial B.P. (mm/Hg) |
| 1 | 10 | 1 | -48 ± 4(n=4) | 275 ± 7 |
| | | 2 | -49 ± 6(n=3) | |
| | | 4 | -42 ± 4(n=6) | |
| | | 6 | -30 ± 4(n=6) | |
| 3 | 1 | 1 | -33 ± 5 (n=5) | 276 ± 6 |
| | | 2 | -17 ± 3 (n=4) | |
| | | 4 | -22 ± 4 (n=6) | |
| | | 6 | -19 ± 5 (n=6) | |
| 5 | 1 | 1 | -40 (n=1) | 262 ± 7 |
| | | 2 | -43 (n=1) | |
| | | 4 | -43 ± 4 (n=1) | |
| | | 6 | -31 ± 5 (n=5) | |
| 6 | 1 | 1 | -18 ± 3 | 276 ± 7 |
| | | 2 | -18 ± 3 | |
| | | 4 | -13 ± 3 | |
| | | 6 | - 9 ± 2 | |
| 7 | 1 | 1 | -31 ± 3 | 244 ± 8 |
| | | 2 | -22 ± 4 | |
| | | 4 | -20 ± 4 | |
| | | 6 | -12 ± 6 | |
| 8 | 1 | 1 | -12 ± 5 | 269 ± 11 |
| | | 2 | -15 ± 3 | |
| | | 4 | -26 ± 8 | |
| | | 6 | -21 ± 2 | |
| 9 | 3 | 1 | -12 ± 2 | 238 ± 8 |
| | | 2 | -16 ± 3 | |
| | | 4 | -14 ± 4 | |
| | | 6 | -11 ± 2 | |

Toxicology

No toxicological effects were indicated in any of the abovementioned tests.

## Claims

1. A compound of formula (I):

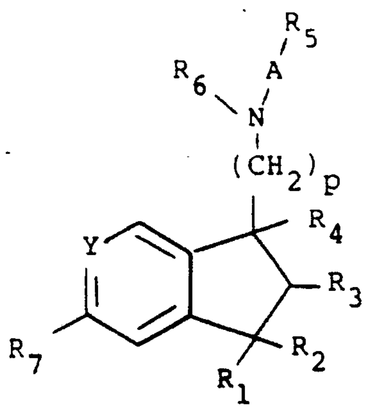

(I)

or, where appropriate, a pharmaceutically acceptable salt thereof, or a pharmaceutically acceptable solvate thereof
characterised in that:
A represents >C=X, wherein
X represents oxygen or sulphur, or A-represents a bond;
Y represents N or $N^+-O^-$ or a moiety $CR_8$ wherein
$R_8$ is as defined below;
$R_1$ and $R_2$ independently represent hydrogen or alkyl; or
$R_1$ and $R_2$ together represent a $C_{2-7}$ polymethylene moiety;
$R_3$ represents hydrogen, hydroxy, alkoxy or acyloxy and $R_4$ is hydrogen or $R_3$ and $R_4$ together represent a bond;
when A represents >C=X, then $R_5$ is hydrogen;
alkyl optionally substituted by one or more groups or atoms selected from halogen, hydroxy, alkoxy, alkoxycarbonyl, carboxy or an ester or amide thereof, amino, monoalkylamino or dialkylamino; alkenyl; amino optionally substituted by an alkyl or alkenyl group or by an alkanoyl group optionally substituted by up to three halo atoms, by a phenyl group optionally substituted by alkyl, alkoxy or halogen; substituted or unsubstituted aryl or substituted or unsubstituted heteroaryl; and
$R_6$ represents hydrogen or alkyl;
or $R_5$ and $R_6$ together represent a linking chain of formula $-A^1-A^2-$, $A^1$ being attached to the nitrogen atom of the moiety -N-A- and $A^2$ being attached to the group A on the said moiety, and wherein $A^1$ represents a substituted or unsubstituted methylene group, $A^2$ represents 2 or 3 linking members, one of the linking members optionally representing O, S or NR and the other linking members each independently representing a substituted or unsubstituted methylene group; R represents hydrogen, alkyl, alkanoyl, phenyl $C_{1-4}$-alkyl, arylcarbonyl wherein the aryl group may be substituted or unsubstituted; or R is mono- or bi-cyclic-heteroarylcarbonyl; when A represents a bond, then $R_5$ and $R_6$ together with the nitrogen atom to which they are attached, form an unsaturated heterocyclic ring having 5 to 7 ring atoms, which ring atoms comprise up to 2 further nitrogen atoms and a carbon atom, the carbon atom being substituted with either an oxo group or a thioxo group the remaining ring atoms being substituted or unsubstituted;
$R_7$ and $R_8$ are each independently selected from the class of hydrogen, substituted or unsubstituted alkyl, alkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$-, $R^dCS.O$-, $R^d(OH)CH$-, $R^d(SH)CH$-, $R^dC(=N.OH)$-, $R^dC(=N.NH_2)$- or alkenyl optionally substituted by alkylcarbonyl, nitro or cyano, providing that when Y is $CR_8$ then at least one of $R_7$ or $R_8$ is not hydrogen;
$R^a$ represents $R^d$ or $R^dO$- and $R^d$ represents substituted or unsubstituted alkyl, substituted or unsubstituted aryl or substituted or unsubstituted heteroaryl optional substituents for $R^d$ being up to 3 substituents selected from alkyl, alkoxy, halo, haloalkyl, nitro and cyano;
$R^b$ and $R^c$ each independently represent hydrogen, alkyl or alkylcarbonyl;
T represents a bond or $T^1$, $T^1$ represents -CS- or $T^2$ and $T^2$ represents -CO-, -SO- or $-SO_2$-; and
p represents zero or an integer 1.

2. A compound according to claim 1, wherein Y is $CR_8$ , one of $R_7$ and $R_8$ is hydrogen and the other is selected from substituted or unsubstituted alkyl, alkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$-, $R^dCS.O$-, $R^d(OH)CH$-, $R^d(SH)CH$-, $R^dC(=N.OH)$-, $R^dC(=N.NH_2)$- or alkenyl optionally substituted by alkylcarbonyl, nitro or cyano, wherein $R^a$, $R^b$, $R^c$, T, $T^1$ and $T^2$ are as defined in relation to formula (I).

3. A compound according to claim 1 or claim 2, wherein one of $R_7$ and $R_8$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkyl-thiocarbonyl, $C_{1-6}$ alkoxy-thiocarbonyl, $C_{1-6}$ alkyl-thiocarbonyloxy, $C_{1-6}$ alkyl-thiomethyl, formul or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino $C_{1-6}$ alkoxysulphinylamino or $C_{1-6}$ alkoxysulphonylamino or ethenyl terminally substituted by $C_{1-6}$ alkylcarbonyl, nitro or cyano, or -C($C_{1-6}$ alkyl)NOH or -C($C_{1-6}$ alkyl)$NNH_2$.

4. A compound according to any one of claims 1 to 3, wherein:
A represents >C=X, wherein
X represents oxygen or sulphur, or A represents a bond;
Y represents N or $N^+$-$O^-$ or a moiety $CR_8$ wherein
$R_8$ is as defined below;
$R_1$ and $R_2$ independently represent hydrogen or $C_{1-6}$ alkyl; or $R_1$ and $R_2$ together represent a $C_{2-7}$ polymethylene moiety;
$R_3$ represents hydrogen, hydroxy, $C_{1-6}$ alkoxy or $C_{1-7}$ acyloxy and $R_4$ is hydrogen or $R_3$ and $R_4$ together represent a bond;
when A represents >C=X, then $R_5$ is hydrogen; $C_{1-6}$ alkyl optionally substituted by halogen, hydroxy, $C_{1-6}$ alkoxy, $C_{1-6}$ alkoxycarbonyl, carboxy or amino optionally substituted by one or two independent $C_{1-6}$ alkyl groups; or $C_{2-6}$ alkenyl; amino optionally substituted by a $C_{1-6}$ alkyl or $C_{2-6}$ alkenyl group or by a $C_{1-6}$ alkanoyl group optionally substituted by up to three halo atoms, by a phenyl group optionally substituted by $C_{1-6}$ alkyl, $C_{1-6}$ alkoxy or halogen; or aryl or heteroaryl, either being optionally substituted by one or more groups or atoms selected from the class of $C_{1-6}$ alkyl, $C_{1-6}$ alkoxy, hydroxy, halogen, trifluoromethyl, nitro, cyano $C_{1-12}$ carboxylic acyl, or amino or aminocarbonyl optionally substituted by one or two $C_{1-6}$ alkyl groups; and
$R_6$ represents hydrogen or $C_{1-6}$ alkyl;
or $R_5$ and $R_6$ together represent $-CH_2-(CH_2)_n-Z-(CH_2)_m-$wherein m and n are 0 to 2 such that m + n is 1 or 2 and Z is $CH_2$, O, S or NR wherein R is hydrogen, $C_{1-9}$ alkyl, $C_{2-7}$ alkanoyl, phenyl $C_{1-4}$- alkyl, naphthylcarbonyl, phenylcarbonyl or benzylcarbonyl optionally substituted in the phenyl or naphthyl ring by one or two of $C_{1-6}$ alkyl, $C_{1-6}$ alkoxy or halogen; or R is mono- or bi-cyclic- heteroarylcarbonyl;
when A represents a bond, then $R_5$ and $R_6$ together with the nitrogen atom to which they are attached, form an unsaturated heterocyclic ring having 5 to 7 ring atoms, which ring atoms comprise up to 2 further nitrogen atoms and a carbon atom, the carbon atom being substituted with either an oxo group or a thioxo group the remaining ring atoms being substituted or unsubstituted;
when Y is N or $N^+$-$O^-$, $R_7$ is hydrogen or, when Y is $CR_8$, either one of $R_7$ and $R_8$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkyl-thiocarbonyl, $C_{1-6}$ alkoxy-thiocarbonyl, $C_{1-6}$ alkyl-thiocarbonyloxy, $C_{1-6}$ alkyl-thiomethyl, formyl or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino $C_{1-6}$ alkoxysulphinylamino or $C_{1-6}$ alkoxysulphonylamino or ethenyl terminally substituted by $C_{1-6}$ alkylcarbonyl, nitro or cyano, or -C-($C_{1-6}$ alkyl)NOH or -C($C_{1-6}$ alkyl)$NNH_2$; or one of $R_7$ and $R_8$ is nitro, cyano or $C_{1-3}$ alkylcarbonyl and the other is methoxy or amino optionally substituted by one or two $C_{1-6}$ alkyl or by $C_{2-7}$ alkanoyl; or $R_7$ is hydrogen and $R_8$ is $C_{1-6}$ alkyl or $C_{3-8}$ cycloalkyl.

5. A compound according to any one of claims 1 to 3, wherein $R_5$ and $R_6$ together represent a linking chain $-A^1-A^2-$, $A^1$ represents a substituted or unsubstituted methylene group and $A^2$ represents a $-CH_2CH_2-$ or $-CH_2CH_2CH_2-$ group.

6. A compound according to any one of claims 1 to 3 wherein $R_5$ and $R_6$ together represent the moiety $-CH_2-(CH_2)_n-Z-(CH_2)_m$.

7. A compound according to any one of claims 1 to 6 wherein the moiety $R_5$.N.CX.$R_6$ represents either pyrrolidonyl or piperidonyl.

8. A compound according to any one of claims 1 to 6, wherein the unsaturated, heterocyclic ring represented by the moiety $R_6$.N.$R_5$ includes 5- or 6- ring atoms.

9. A compound according to any one of claims 1 to 6, wherein A represents a bond and the moiety $R_6$.N.$R_5$ represents a substituted or unsubstituted pyridonyl or substituted or unsubstituted thiopyridonyl group.

10. A compound according to claim 9, wherein the pyridonyl group is a 2-pyridon-1-yl group.

11. A compound according to any one of claims 1 to 10, wherein p represents zero.

12. A compound according to claim 1, selected from the group consisting of:
cis 1,1-dimethyl-5-nitro-3-(2-oxopyrrolidin-1-yl)indan-2-ol;
trans 1,1-dimethyl-5-nitro-3-(2-oxopyrrolidin-1-yl)indan- 2-ol;
trans 1,1-dimethyl-5-nitro-3-(2-oxopiperidin-1-yl) indan-2-ol;
1,1-dimethyl-5-nitro-3-(2-oxopiperidin-1-yl)ind-2-ene;
1,1-dimethyl-5-nitro-3-(2-pyridon-1-yl)ind-2-ene;
trans-1,1-dimethyl-5-nitro-3-(2-pyridon-1-yl)indan-2-ol;
trans-5-cyano-1,1-dimethyl-3-(2-pyridon-1-yl)-indan-2-ol;
5-cyano-1,1-dimethyl-3-(2-pyridon-1-yl)ind-2-ene;
trans-5-amino-1,1-dimethyl-3-(2-pyridon-1-yl)indan-2-ol;
trans-1-N-(4-fluorobenzamido)-6-nitroindan-2-ol;
trans-1-N-(4-chlorobutyrylamido)-6-nitroindan-2-ol;
trans-6-nitro-1-(2-oxopyrrolidin-1-yl)indan-2-ol;
trans-3-(4-fluorobenzoyl-N-methylamino)-1,1-dimethyl-5-nitroindan-2-ol;
trans-3-(4-fluorobenzoyl-N-methylamino)-1,1-dimethyl-5-cyanoindan-2-ol;
trans-1,1-dimethyl-3-(2-oxopiperidin-1-yl)-6-trifluoromethylindan-2-ol;
1,1-dimethyl-3-(2-oxopiperidin-1-yl)-6-trifluoromethyl-ind-2-ene;
trans-1,1-dimethyl-5-ethyl-3-(2-oxopiperidin-1-yl)indan -2-ol;
trans-1,1-dimethyl-6-pentafluoroethyl-3-(2-oxo-piperidin-1-yl)indan-2-ol;
1,1-dimethyl-6-pentafluoroethyl-3-(2-oxopiperidin-1-yl)ind-2-ene;
trans-1,1-dimethyl-3-(2-pyridon-1-yl)-6-trifluoromethyl indan-2-ol;
trans-1,1-dimethyl-6-ethyl-3-(2-oxopiperidin-1-yl)indan -2-ol;
trans-6-cyano-1,1-dimethyl-3-(2-oxopiperidin-1-yl)indan -2-ol;
trans-1,1-dimethyl-5-ethyl-3-(2-pyridon-1-yl)indan-2-ol;
5-cyano-1,1-dimethyl-3-[(2-oxopyrrolidin-1-yl)methyl] ind-2-ene;
5-cyano-1,1-dimethyl-3-[(2-pyridon-1-yl)methyl] ind-2-ene;
trans-1,1-dimethyl-5-nitro-3-(5-(R)-methyl-2-oxopyrrolidin-1-yl)indan-2-ol;
1,1-dimethyl-5-nitro-3-(5-(R)-methyl-2-oxopyrrolidin-1-yl)ind-2-ene;
trans-1,1-dimethyl-5-nitro-3-(5-(S)-methyl-2-oxopyrrolidin-1-yl)indan-2-ol;
1,1-dimethyl-5-nitro-3-(5-(S)-methyl-2-oxopyrrolidin-1-yl)ind-2-ene; and
trans-1,1-dimethyl-5-iodo-3-(2-oxopiperidin-1-yl)indan-2-ol or a pharmaceutically acceptable salt thereof or a pharmaceutically acceptable solvate thereof.

13. A process for the preparation of a compound of formula (I) or, where appropriate a pharmaceutically acceptable salt thereof, or a pharmaceutically acceptable solvate thereof, which comprises;
i) for compounds of formula (I) wherein p represents zero and A represents >C=X, acylating a compound of formula (II):

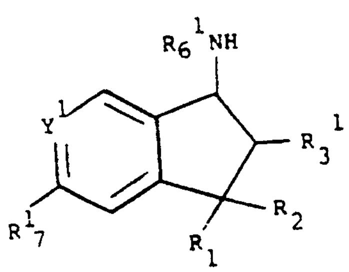

(II)

wherein, $Y^1$ represents Y or a group convertible to Y, $R_1$ and $R_2$ are as defined hereinbefore, $R_3{}^1$ is hydroxy, alkoxy or acyloxy, $R_6{}^1$ is hydrogen or alkyl and $R_7{}^1$ represents $R_7$ or a group or atom convertible

to $R_7$,
a) with an acylating agent of formula (III):
$R_{10}$-CO-$L_1$ (III)
wherein $L_1$ is a leaving group, and $R_{10}$ is hydrogen, substituted or unsubstituted alkyl wherein the substituents are as hereinbefore defined for $R_5$; amino optionally substituted as hereinbefore defined for $R_5$; alkenyl or optionally substituted aryl or heteroaryl as hereinbefore defined for $R_5$, or a group convertible to $R_5$ as hereinbefore defined, and thereafter, when $R_6$ is hydrogen and $R_{10}$ is $X^1CH_2(CH_2)_z$, where z is 2 or 3; and $X^1$ is a leaving group, cyclising the resultant compound;
b) with a compound of formula (IV)
$X=C=N.R_{11}$ (IV)
wherein $R_{11}$ is hydrogen, alkyl, alkenyl, alkanoyl, optionally substituted by up to three halo atoms, or phenyl optionally substituted by alkyl, alkoxy or halogen; and X is oxygen or sulphur, and thereafter when $R_{11}$ is hydrogen, optionally converting $R_{11}$; or
ii) for compounds of formula (I) wherein p represents zero and A represents >C=X and $R_5$ and $R_6$ together represent a linking chain of formula $-A^1-A^2-$ as defined above in relation to formula (I), by reacting a compound of formula (V):

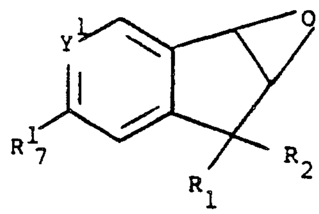

(V)

wherein $R_1$ and $R_2$ are as hereinbefore defined in relation to formula (I) and $R_7{}^1$ and $Y^1$ are as defined in relation to formula (II), with an activated form of a compound of formula (VIA):
$R_{13}NHCOR_{12}$ (VIA)
wherein $R_{12}$ and $R_{13}$ together represents a linking chain of formula $-A^1-A^2-$.
iii) for compounds of formula (I) wherein p represents zero and A represents a bond and $R_5$ and $R_6$ together with the nitrogen to which they are attached, form the above defined unsaturated heterocyclic ring, by reacting a compound of formula (V) as defined above with an activated form of a compound of formula (VIB):
$R_6'NHR_5'$ (VIB)
wherein $R_5'$ and $R_6'$ form an unsaturated heterocyclic ring as defined above in respect of the variables $R_5$ and $R_6$, or
iv) for compounds of formula (I) wherein p represents 1, reacting a compound of formula (VII):

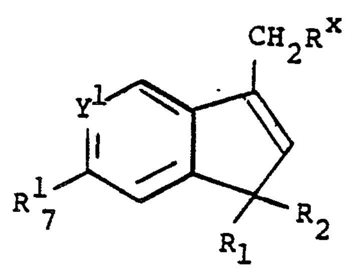

(VII)

wherein $R_1$ and $R_2$ are as defined in relation to formula (I), $Y^1$ represents Y or a group convertible to Y, $R_7{}^1$ represents $R_7$ or a group convertible to $R_7$ and $R^x$ represents a leaving group, with a compound of formula (VIII):
$R_6$-NH-A-$R_5$ (VIII)
or an activated form thereof, wherein $R_5$, $R_6$ and A are as defined in relation to formula (I); or
v) reacting a compound of formula (X), as defined above, with a base to provide a compound of the above defined formula (V) and thereafter reacting the compound of formula (V) <u>in situ</u> with either a compound of formula (VIA) or (VIB) as respectively described above in reaction ii) or reaction iii):

and thereafter if required, carrying out one or more of the following optional steps:

(a) converting a compound of formula (I) into a further compound of formula (I);

(b) converting $Y^1$ to Y;

(c) converting $R_7{}^1$ to $R_7$;

(d) forming a pharmaceutically acceptable salt of the compound of formula (I);

(e) forming a pharmaceutically acceptable solvate of the compound of formula (I) or a pharmaceutically acceptable salt thereof.

14. A pharmaceutical composition which comprises a compound of formula (I), or a pharmaceutically acceptable salt thereof or a pharmaceutically acceptable solvate thereof, and a pharmaceutically acceptable carrier.

15. A compound of formula (I) or a pharmaceutically acceptable salt thereof, or a pharmaceutically acceptable solvate thereof, for use in the treatment of respiratory tract disorders or hypertension or disorders associated with smooth muscle contraction of the gastro intestinal tract or the abovementioned cardiovascular disorders.

16. The use of a compound of formula (I), or a pharmaceutically acceptable salt thereof, or a pharmaceutically acceptable solvate thereof for the manufacture of a medicament for the treatment of respiratory tract disorders or hypertension or disorders associated with smooth muscle contraction of the gastro intestinal tract or the abovementioned cardiovascular disorders.

Claims for the following Contracting State: ES

1. A process for preparing a compound of formula (I):

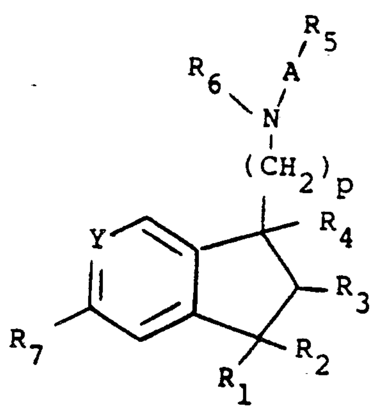

(I)

or, where appropriate, a pharmaceutically acceptable salt thereof, or a pharmaceutically acceptable solvate thereof

wherein:

A represents >C=X, wherein

X represents oxygen or sulphur, or A represents a bond;

Y represents oxygen or sulphur, or A represents a bond;

Y represents N or $N^+$-$O^-$ or a moiety $CR_8$ wherein

$R_8$ is as defined below;

$R_1$ and $R_2$ independently represent hydrogen or alkyl; or

$R_1$ and $R_2$ together represent a $C_{2-7}$ polymethylene moiety;

$R_3$ represents hydrogen, hydroxy, alkoxy or acyloxy and $R_4$ is hydrogen or $R_3$ and $R_4$ together represent a bond;

when A represents >C=X, then $R_5$ is hydrogen;

alkyl optionally substituted by one or more groups or atoms selected from halogen, hydroxy, alkoxy, alkoxycarbonyl, carboxy or an ester or amide thereof, amino, monoalkylamino or dialkylamino; alkenyl; amino optionally substituted by an alkyl or alkenyl group or by an alkanoyl group optionally substituted by up to three halo atoms, by a phenyl group optionally substituted by alkyl, alkoxy or halogen; substituted or unsubstituted aryl or substituted or unsubstituted heteroaryl; and

$R_6$ represents hydrogen or alkyl;

or $R_5$ and $R_6$ together represent a linking chain of formula $-A^1-A^2-$, $A^1$ being attached to the nitrogen atom of the moiety -N-A- and $A^2$ being attached to the group A on the said moiety, and wherein $A^1$ represents a substituted or unsubstitited methylene group, $A^2$ represents 2 or 3 linking members, one of the linking members optionally representing O, S or NR and the other linking members each independently representing a substituted or unsubstituted methylene group; R represents hydrogen, alkyl, alkanoyl, phenyl $C_{1-4}$-alkyl, arylcarbonyl wherein the aryl group may be substituted or unsubstituted; or R is mono- or bi-cyclic-heteroarylcarbonyl; when A represents a bond, then $R_5$ and $R_6$ together with the nitrogen atom to which they are attached, form an unsaturated heterocyclic ring having 5 to 7 ring atoms, which ring atoms comprise up to 2 further nitrogen atoms and a carbon atom, the carbon atom being substituted with either an oxo group or a thioxo group the remaining rings atoms being substituted or unsubstituted;

$R_7$ and $R_8$ are each independently selected from the class of hydrogen, substituted or unsubstituted alkyl, alkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$-, $R^dCS.O$-, $R^d(OH)CH$-, $R^d(SH)CH$-, $R^dC(=N.OH)$-, $R^dC(=N.NH_2)$- or alkenyl optionally substituted by alkylcarbonyl, nitro or cyano, providing that when Y is $CR_8$ then at least one of $R_7$ or $R_8$ is not hydrogen;

$R^a$ represents $R^d$ or $R^dO$- and $R^d$ represents substituted or unsubstituted alkyl, substituted or unsubstituted aryl or substituted or unsubstituted heteroaryl, optional substituents for $R^d$ being up to 3 substituents selected from alkyl, alkoxy, halo, haloalkyl, nitro and cyano;

$R^b$ and $R^c$ each independently represent hydrogen, alkyl or alkylcarbonyl;

T represents a bond or $T^1$, $T^1$ represents -CS- or $T^2$ and $T^2$ represents -CO-, -SO- or $-SO_2-$; and

p represents zero or an integer 1,

which process comprises:

i) for compounds of formula (I) wherein p represents zero and A represents >C=X, acylating a compound of formula (II):

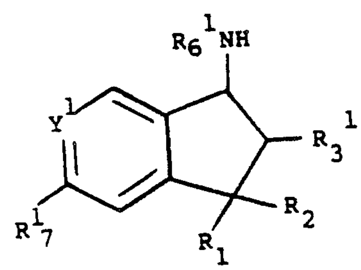

(II)

wherein, $Y^1$ represents Y or a group convertible to Y, $R_1$ and $R_2$ are as defined hereinbefore, $R_3{}^1$ is hydroxy, alkoxy or acyloxy, $R_6{}^1$ is hydrogen or alkyl and $R_7{}^1$ represents $R_7$ or a group or atom convertible to $R_7$,

a) with an acylating agent of formula (III):

$R_{10}$-CO-$L_1$ (III)

wherein $L_1$ is a leaving group, and $R_{10}$ is hydrogen, substituted or unsubstituted alkyl wherein the substituents are as hereinbefore defined for $R_5$; amino optionally substituted as hereinbefore defined for $R_5$; alkenyl or optionally substituted aryl or heteroaryl as hereinbefore defined for $R_5$, or a group convertible to $R_5$ as hereinbefore defined, and thereafter, when $R_6$ is hydrogen and $R_{10}$ is $X^1CH_2(CH_2)_z$, where z is 2 or 3; and $X^1$ is a leaving group, cyclising the resultant compound;

b) with a compound of formula (IV)

$X=C=N.R_{11}$ (IV)

wherein $R_{11}$ is hydrogen, alkyl, alkenyl, alkanoyl optionally substituted by up to three halo atoms, or phenyl optionally substituted by alkyl, alkoxy or halogen; and X is oxygen or sulphur, and thereafter then $R_{11}$ is hydrogen, optionally converting $R_{11}$; or

ii) for compounds of formula (I) wherein p represents zero and A represents >C=X and $R_5$ and $R_6$ together represent a linking chain of formula $-A^1-A^2-$ as defined above in relation to formula (I), by reacting a compound of formula (V):

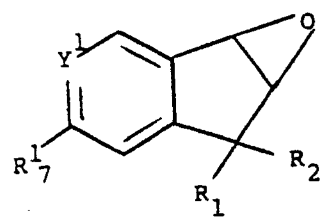

(V)

wherein $R_1$ and $R_2$ are as hereinbefore defined in relation to formula (I) and $R_7{}^1$ and $Y^1$ are as defined in relation to formula (II), with an activated form of a compound of formula (VIA):

$R_{13}NHCOR_{12}$ (VIA)

wherein $R_{12}$ and $R_{13}$ together represents a linking chain of formula $-A^1-A^2-$.

iii) for compounds of formula (I) wherein p represents zero and A represents a bond and $R_5$ and $R_6$ together with the nitrogen to which they are attached, form the above defined unsaturated heterocyclic ring, by reacting a compound of formula (V) as defined above with an activated form of a compound of formula (VIB):

$R_6'NHR_5'$ (VIB)

wherein $R_5'$ and $R_6'$ form an unsaturated heterocyclic ring as defined above in respect of the variables $R_5$ and $R_6$, or

iv) for compounds of formula (I) wherein p represents 1, reacting a compound of formula (VII):

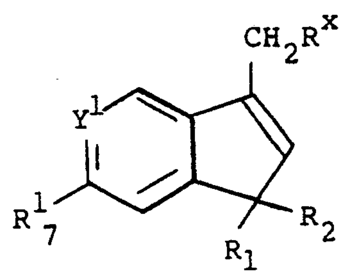

(VII)

wherein $R_1$ and $R_2$ are as defined in relation to formula (I), $Y^1$ represents Y or a group convertible to Y, $R_7{}^1$ represents $R_7$ or a group convertible to $R_7$ and $R^x$ represents a leaving group, with a compound of formula (VIII):

$R_6$-NH-A-$R_5$ (VIII)

or an activated form thereof, wherein $R_5$, $R_6$ and A are as defined in relation to formula (I); or

v) reacting a compound of formula (X), as defined above, with a base to provide a compound of the above defined formula (V) and thereafter reacting the compound of formula (V) in situ with either a compound of formula (VIA) or (VIB) as respectively described above in reaction ii) or reaction iii):

and thereafter if required, carrying out one or more of the following optional steps:

(a) converting a compound of formula (I) into a further compound of formula (I);

(b) converting $Y^1$ to Y;

(c) converting $R_7{}^1$ to $R_7$;

(d) forming a pharmaceutically acceptable salt of the compound of formula (I);

(e) forming a pharmaceutically acceptable solvate of the compound of formula (I) or a pharmaceutically acceptable salt thereof.

2. A process according to claim 1 or claim 2, for preparing a compound of formula (I) wherein Y is $CR_8$, one of $R_7$ and $R_8$ is hydrogen and the other is selected from substituted or unsubstituted alkyl, alkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$-, $R^dCS.O$-, $R^d(OH)CH$-, $R^d(SH)CH$-, $R^dC(=N.OH)$-, $R^dC(=N.NH_2)$- or alkenyl optionally substituted by alkylcarbonyl, nitro or cyano, wherein $R^a$, $R^b$, $R^c$, T, $T^1$ and $T^2$ are as defined in relation to formula (I).

3. A process according to claim 1 or claim 2, for preparing a compound of formula (I) wherein one of $R_7$ and $R_8$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, alkoxycarbonylamino, $C_{1-6}$ alkyl-thiocarbonyl, $C_{1-6}$ alkoxy-thiocarbonyl, $C_{1-6}$ alkyl-thiocarbonyloxy, $C_{1-6}$ alkylthiomethyl, formyl or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino $C_{1-6}$ alkoxysulphinylamino or $C_{1-6}$ alkoxysulphonylamino or ethenyl terminally substituted by $C_{1-6}$ alkylcarbonyl, nitro or cyano, or -C($C_{1-6}$ alkyl)NOH or -C($C_{1-6}$ alkyl)$NNH_2$.

4. A process according to any one of claims 1 to 3, for preparing a compound of formula (I) wherein:
A represents >C=X, wherein
X represents oxygen or sulphur, or A represents a bond;
Y represents N or $N^+$-$O^-$ or a moiety $CR_8$ wherein
$R_8$ is as defined below;
$R_1$ and $R_2$ independently represent hydrogen or $C_{1-6}$ alkyl; or $R_1$ and $R_2$ together represent a $C_{2-7}$ polymethylene moiety;
$R_3$ represents hydrogen, hydroxy, $C_{1-6}$ alkoxy or $C_{1-7}$ acyloxy and $R_4$ is hydrogen or $R_3$ and $R_4$ together represent a bond;
when A represents >C=X, then $R_5$ is hydrogen; $C_{1-6}$ alkyl optionally substituted by halogen, hydroxy, $C_{1-6}$ alkoxy, $C_{1-6}$ alkoxycarbonyl, carboxy or amino optionally substituted by one or two independent $C_{1-6}$ alkyl groups; or $C_{2-6}$ alkenyl; amino optionally substituted by a $C_{1-6}$ alkyl or $C_{2-6}$ alkenyl group or by a $C_{1-6}$ alkanoyl group optionally substituted by up to three halo atoms, by a phenyl group optionally substituted by $C_{1-6}$ alkyl, $C_{1-6}$ alkoxy or halogen; or aryl or heteroaryl, either being optionally substituted by one or more groups or atoms selected from the class of $C_{1-6}$ alkyl, $C_{1-6}$ alkoxy, hydroxy, halogen, trifluoromethyl, nitro, cyano, $C_{1-12}$ carboxylic acyl, or amino or aminocarbonyl optionally substituted by one or two $C_{1-6}$ alkyl groups; and
$R_6$ represents hydrogen or $C_{1-6}$ alkyl;
or $R_5$ and $R_6$ together represent $-CH_2-(CH_2)_n-Z-(CH_2)_m-$wherein m and n are 0 to 2 such that m + n is 1 or 2 and Z is $CH_2$, O, S or NR wherein R is hydrogen, $C_{1-9}$ alkyl, $C_{2-7}$ alkanoyl, phenyl $C_{1-4}$- alkyl, naphthylcarbonyl, phenylcarbonyl or benzylcarbonyl optionally substituted in the phenyl or naphthyl ring by one or two of $C_{1-6}$ alkyl, $C_{1-6}$ alkoxy or halogen; or R is mono- or bi-cyclic- heteroarylcarbonyl;
when A represents a bond, then $R_5$ and $R_6$ together with the nitrogen atom to which they are attached, form an unsaturated heterocyclic ring having 5 to 7 ring atoms, which ring atoms comprise up to 2 further nitrogen atoms and a carbon atom, the carbon atom being substituted with either an oxo group or a thioxo group the remaining ring atoms being substituted or unsubstituted;
when Y is N or $N^+$-$O^-$, $R_7$ is hydrogen or, when Y is $CR_8$, either one of $R_7$ and $R_8$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkylthiocarbonyl, $C_{1-6}$ alkoxy-thiocarbonyl, $C_{1-6}$ alkyl-thiocarbonyloxy, $C_{1-6}$ alkyl-thiomethyl, formyl or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino $C_{1-6}$ alkoxysulphinylamino or $C_{1-6}$ alkoxysulphonylamino or ethenyl terminally substituted by $C_{1-6}$ alkylcarbonyl, nitro or cyano, or -C-($C_{1-6}$ alkyl)NOH or -C($C_{1-6}$ alkyl)$NNH_2$; or one of $R_7$ and $R_8$ is nitro, cyano or $C_{1-3}$ alkylcarbonyl and the other is methoxy or amino optionally substituted by one or two $C_{1-6}$ alkyl or by $C_{2-7}$ alkanoyl; or $R_7$ is hydrogen and $R_8$ is $C_{1-6}$ alkyl or $C_{3-8}$ cycloalkyl.

5. A process according to any one of claims 1 to 3, for preparing a compound of formula (I) wherein $R_5$ and $R_6$ together represent a linking chain $-A^1-A^2-$, $A^1$ represents a substituted or unsubstituted methylene group and $A^2$ represents a $-CH_2CH_2-$ or $-CH_2CH_2CH_2-$group.

6. A process according to any one of claims 1 to 3, for preparing a compound of formula (I) wherein $R_5$ and $R_6$ together represent the moiety $-CH_2-(CH_2)_n-Z-(CH_2)_m$.

7. A process according to any one of claims 1 to 6, for preparing a compound of formula (I) wherein the moiety $R_5$.N.CX.$R_6$ represents either pyrrolidonyl or piperidonyl.

8. A process according to any one of claims 1 to 6, for preparing a compound of formula (I) wherein the unsaturated, heterocyclic ring represented by the moiety $R_6$.N.$R_5$ includes 5- or 6- ring atoms.

9. A process according to any one of claims 1 to 6, for preparing a compound of formula (I) wherein A represents a bond and the moiety $R_6$.N.$R_5$ represents a substituted or unsubstituted pyridonyl or substituted or unsubstituted thiopyridonyl group.

10. A process according to claim 9, for preparing a compound of formula (I) wherein the pyridinyl group is a 2-pyridon-1-yl group.

11. A process according to any one of claims 1 to 10, for preparing a compound of formula (I) wherein p represents zero.

12. A process according to claim 1, for preparing a compound of formula (I) selected from the group consisting of:
cis 1,1-dimethyl-5-nitro-3-(2-oxopyrrolidin-1-yl)indan-2-ol;
trans 1,1-dimethyl-5-nitro-3-(2-oxopyrrolidin-1-yl)indan- 2-ol;
trans 1,1-dimethyl-5-nitro-3-(2-oxopiperidin-1-yl)indan-2-ol;
1,1-dimethyl-5-nitro-3-(2-oxopiperidin-1-yl)ind-2-ene;
1,1-dimethyl-5-nitro-3-(2-pyridon-1-yl)ind-2-ene;
trans-1,1-dimethyl-5-nitro-3-(2-pyridon-1-yl)indan-2-ol;
trans-5-cyano-1,1-dimethyl-3-(2-pyridon-1-yl)-indan-2-ol;
5-cyano-1,1-dimethyl-3-(2-pyridon-1-yl)ind-2-ene;
trans-5-amino-1,1-dimethyl-3-(2-pyridon-1-yl)indan-2-ol;
trans-1,N-(4-fluorobenzamido)-6-nitroindan-2-ol;
trans-1-N-(4-chlorobutyrylamido)-6-nitroindan-2-ol;
trans-6-nitro-1-(2-oxopyrrolidin-1-yl)indan-2-ol;
trans-3-(4-fluorobenzoyl-N-methylamino)-1,1-dimethyl-5-nitroindan-2-ol;
trans-3-(4-fluorobenzoyl-N-methylamino)-1,1-dimethyl-5-cyanoindan-2-ol;
trans-1,1-dimethyl-3-(2-oxopiperidin-1-yl)-6-trifluoromethylindan-2-ol;
1,1-dimethyl-3-(2-oxopiperidin-1-yl)-6-trifluoromethylind-2-ene;
trans-1,1-dimethyl-5-ethyl-3-(2-oxopiperidin-1-yl)indan-2-ol;
trans-1,1-dimethyl-6-pentafluoroethyl-3-(2-oxopiperidin-1-yl)indan-2-ol;
1,1-dimethyl-6-pentafluoroethyl-3-(2-oxopiperidin-1-yl)ind-2-ene;
trans-1,1-dimethyl-3-(2-pyridon-1-yl)-6-trifluoromethyl indan-2-ol;
trans-1,1-dimethyl-6-ethyl-3-(2-oxopiperidin-1-yl)indan-2-ol;
trans-6-cyano-1,1-dimethyl-3-(2-oxopiperidin-1-yl)indan-2-ol;
trans-1,1-dimethyl-5-ethyl-3-(2-pyridon-1-yl)indan-2-ol;
5-cyano-1,1-dimethyl-3-[(2-oxopyrrolidin-1-yl)methyl] ind-2-ene;
5-cyano-1,1-dimethyl-3-[(2-pyridon-1-yl)methyl] ind-2-ene;
trans-1,1-dimethyl-5-nitro-3-(5-(R)-methyl-2-oxopyrrolidin-1-yl)indan-2-ol;
1,1-dimethyl-5-nitro-3-(5-(R)-methyl-2-oxopyrrolidin-1-yl)ind-2-ene;
trans-1,1-dimethyl-5-nitro-3-(5-(S)-methyl-2-oxopyrrolidin-1-yl)indan-2-ol;
1,1-dimethyl-5-nitro-3-(5-(S)-methyl-2-oxopyrrolidin-1-yl)ind-2-ene; and
trans-1,1-dimethyl-5-iodo-3-(2-oxopiperidin-1-yl)indan-2-ol or a pharmaceutically acceptable salt thereof or a pharmaceutically acceptable solvate thereof.

13. A compound of formula (I) or a pharmaceutically acceptable salt thereof, or a pharmaceutically acceptable solvate thereof, for use in the treatment of respiratory tract disorders or hypertension or disorders associated with smooth muscle contraction of the gastro intestinal tract or the abovementioned cardiovascular disorders.

14. The use of a compound of formula (I), or a pharmaceutically acceptable salt thereof, or a pharmaceutically acceptable solvate thereof for the manufacture of a medicament for the treatment of respiratory tract disorders or hypertension or disorders associated with smooth muscle contraction of the gastro intestinal tract or the abovementioned cardiovascular disorders.

European Patent Office

# EUROPEAN SEARCH REPORT

Application number

EP 88311762.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 104, no. 5, February 3, 1986, Columbus, Ohio, USA<br>BOGESO K. P. et al. "3-Phenyl-1-indanamines"<br>page 524, column 1, abstract-no. 33 821n<br>& J. Med. Chem., 28 (12), 1817-28 (1985)<br>---- | 1,13, 14 | C 07 D 211/76<br>C 07 D 207/263<br>C 07 D 213/64<br>C 07 C 103/78<br>C 07 C 103/38<br>A 61 K 31/445<br>A 61 K 31/40<br>A 61 K 31/44<br>A 61 K 31/165 |

TECHNICAL FIELDS SEARCHED (Int. Cl.4)

C 07 D 211/00
C 07 D 207/00
C 07 D 213/00
C 07 D 103/00

The present search report has been drawn up for all claims

| Place of search | Date of completion of the search | Examiner |
|---|---|---|
| VIENNA | 22-03-1989 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document